(19) Europäisches Patentamt / European Patent Office / Office européen des brevets

(11) **EP 3 714 067 B1**

(12) **EUROPÄISCHE PATENTSCHRIFT**

(45) Veröffentlichungstag und Bekanntmachung des Hinweises auf die Patenterteilung:
**13.11.2024   Patentblatt 2024/46**

(21) Anmeldenummer: **18804613.0**

(22) Anmeldetag: **19.11.2018**

(51) Internationale Patentklassifikation (IPC):
***C12Q 1/6883*** *(2018.01)*

(52) Gemeinsame Patentklassifikation (CPC):
**C12Q 1/6883;** C12Q 2600/112; C12Q 2600/118;
C12Q 2600/158

(86) Internationale Anmeldenummer:
**PCT/EP2018/081681**

(87) Internationale Veröffentlichungsnummer:
**WO 2019/101663 (31.05.2019 Gazette 2019/22)**

(54) **VERWENDUNG DES ADIPOQ GENEXPRESSIONSLEVELS ZUR EINORDNUNG EINES PROBANDEN IN RISIKOGRUPPEN BEI DER PROGNOSE ODER DIAGNOSE EINER DIABETES MELLITUS TYP II ERKRANKUNG**

USE OF THE ADIPOQ EXPRESSION LEVEL FOR CLASSIFYING A SUBJECT INTO RISK GROUPS FOR THE PROGNOSIS OR DIAGNOSIS OF DIABETES MELLITUS TYPE 2

UTILISATION DU NIVEAU D'EXPRESSION DU GÈNE ADIPOQ POUR CLASSER UN SUJET DANS DES GROUPES DE RISQUE LORS DU PRONOSTIC OU DU DIAGNOSTIC D'UN DIABÈTE SUCRÉ DE TYPE II

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **24.11.2017   DE 102017127857**

(43) Veröffentlichungstag der Anmeldung:
**30.09.2020   Patentblatt 2020/40**

(73) Patentinhaber: **Lipozyt Marker GmbH**
**28357 Bremen (DE)**

(72) Erfinder:
• **JENSEN, Uwe**
**28357 Bremen (DE)**
• **HIERNEIS, Wolfgang**
**20146 Hamburg (DE)**
• **KLEMKE, Markus**
**28777 Bremen (DE)**
• **THIES, Helge Wilhelm**
**28359 Bremen (DE)**

(74) Vertreter: **Eisenführ Speiser**
**Patentanwälte Rechtsanwälte PartGmbB**
**Postfach 10 60 78**
**28060 Bremen (DE)**

(56) Entgegenhaltungen:
**WO-A2-2017/021815**

• IBIYE OWEI ET AL: "Insulin-sensitive and insulin-resistant obese and non-obese phenotypes: role in prediction of incident pre-diabetes in a longitudinal biracial cohort", BMJ OPEN DIABETES RESEARCH & CARE, vol. 5, no. 1, 1 July 2017 (2017-07-01), pages e000415, XP055532963, DOI: 10.1136/bmjdrc-2017-000415
• H. YAGHOOTKAR ET AL: "Genetic Evidence for a Normal-Weight "Metabolically Obese" Phenotype Linking Insulin Resistance, Hypertension, Coronary Artery Disease, and Type 2 Diabetes", DIABETES, vol. 63, no. 12, 21 July 2014 (2014-07-21), US, pages 4369 - 4377, XP055533095, ISSN: 0012-1797, DOI: 10.2337/db14-0318
• YANG XI ET AL: "HMGA2 promotes adipogenesis by activating C/EBP[beta]-mediated expression of PPAR[gamma]", BIOCHEMICAL AND BIOPHYSICAL RESEARCH COMMUNICATIONS, vol. 472, no. 4, 1 April 2016 (2016-04-01), AMSTERDAM, NL, pages 617 - 623, XP055533352, ISSN: 0006-291X, DOI: 10.1016/j.bbrc.2016.03.015

- MIA LOUISE WESTERLUND: "Classication with Kohonen Self-Organizing Maps", 24 April 2005 (2005-04-24), XP055532367, Retrieved from the Internet <URL:https://notendur.hi.is/benedikt/Courses/Mia_report2.pdf> [retrieved on 20181210]
- WANDERLEY ROCHA DENISE ROSSO TENÓRIO ET AL: "Visceral Adiposity Measurements, Metabolic and Inflammatory Profi le in Obese Patients with and Without Type 2 Diabetes Mellitus: A Crosssectional Analysis", CURRENT DIABETES REVIEWS, BENTHAM SCIENCE PUBLISHERS LTD, NETHERLANDS, vol. 13, no. 1, 1 January 2017 (2017-01-01), pages 11 - 18, XP009509974, ISSN: 1875-6417, DOI: 10.2174/1573399812666151015115924
- LI LI ET AL: "Identification of type 2 diabetes subgroups through topological analysis of patient similarity", SCIENCE TRANSLATIONAL MEDICINE, vol. 7, no. 311, 28 October 2015 (2015-10-28), US, pages 311ra174 - 311ra174, XP055533039, ISSN: 1946-6234, DOI: 10.1126/scitranslmed.aaa9364
- AMALIA GASTALDELLI ET AL: "Role of Adipose Tissue Insulin Resistance in the Natural History of Type 2 Diabetes: Results From the San Antonio Metabolism Study", DIABETES, vol. 66, no. 4, 4 January 2017 (2017-01-04), US, pages 815 - 822, XP055532549, ISSN: 0012-1797, DOI: 10.2337/db16-1167

**Beschreibung**

Sie betrifft ferner ein Verfahren zur Prognose und/oder die Diagnose einer Diabetes mellitus Typ II Erkrankung zur Einordnung eines Probanden in Risikogruppen, wobei das Genexpressionslevel des Gens *ADIPOQ* bestimmt wird und nachfolgend die Einordnung des Probanden unter Berücksichtigung dieses Genexpressionslevels in Risikogruppen erfolgt.

Die Prävalenz von Übergewicht und Adipositas sowie mit ihnen assoziierten Erkrankungen wie, Diabetes mellitus Typ II (T2D) ist ein weltweit fortschreitendes Problem. Laut Weltgesundheitsorganisation (WHO) wird Übergewicht ab einem Body-Mass-Index (BMI) von $\geq 25$ kg/m$^2$ - < 30 kg/m$^2$ und Adipositas ab einem BMI von $\geq 30$ kg/m$^2$ definiert. Adipositas wird in weitere Grade aufgeteilt: Adipositas Grad I (BMI = 30 - 34,9 kg/m$^2$), Adipositas Grad II (BMI= 35 - 39,9 kg/m$^2$), Adipositas Grad III (BMI $\geq 40$ kg/m$^2$) sowie Super-Adipositas (BMI $\geq 50$ kg/m$^2$). Nicht immer ist zwingend ein hoher BMI Ursache an T2D zu erkranken, vielmehr kann der BMI lediglich als ein sehr grober Prädiktor dienen. Auch Normalgewichtige (BMI < 25 kg/m$^2$) erkranken an T2D, die genauen wissenschaftlichen Hintergründe, warum auch Normalgewichtige an Krankheiten erkranken, die eher mit Übergewicht und Adipositas assoziiert sind, sind bis heute unklar.

Tomiyama et. al. (Tomiyama AJ, Hunger JM, Nguyen-Cuu J, Wells C (2016) Misclassification of cardiometabolic health when using body mass index categories in NHANES 2005-2012. Int J Obes (Lond) 40:883-6) zeigten anhand gemessener Blutparameter in einer Studie mit 40420 Teilnehmern, dass 50 % der dort untersuchten Übergewichtigen und 29 % an Adipositas Erkrankten kardio-metabolisch als gesund gelten, was aufgrund einer Einteilung nur anhand des BMIs nicht zu erwarten war. Zudem wiesen 30 % der dort untersuchten Normalgewichtigen einen ungesunden kardiometabolischen Zustand auf. Die gemessenen Blutparameter zeigen aber nur den Jetzt-Zustand der Probanden auf und spiegeln nur einen kurzen, zurückliegenden Zeitraum einer kardio-metabolischen Fehlentwicklung der Probanden wieder. Der HbA1c-Test z.B. gibt Auskunft über den Blutglukose-Spiegel der letzten 4 - 12 Wochen und ab einem HbA1c-Wert von $\geq 6,5$ % gilt man als Diabetiker.

Eine T2D-Erkrankung ist gekennzeichnet durch einen relativen Insulinmangel und/oder einer Insulinresistenz der Gewebe im Körper. Während der Pathogenese von T2D nimmt die Insulinresistenz und -produktion über viele Jahre immer mehr zu, bis zu einem Punkt an dem die Insulin-produzierenden $\beta$-Zellen der Bauchspeicheldrüse aufgrund der jahrelangen Überbeanspruchung dysfunktional werden und die Insulinproduktion drosseln. Dieser Zeitpunkt gilt als Beginn einer T2D-Erkrankung, bis der Diabetiker aber als Diabetiker vom Arzt erkannt wird dauert es ebenfalls durchschnittlich nochmals mehrere Jahre (Pearson J, Powers MA (2006) Systematically initiating insulin: the staged diabetes management approach. Diabetes Educ 32 (Suppl):19S-28S). Im Gegensatz zum Typ-1-Diabetes (T1D), wo die Diabetes-Ursache in einer dysfunktionalen Bauchspeicheldrüse liegt, können beim T2D neben der Bauchspeicheldrüse auch die Leber, Muskeln und/oder Fehlentwicklungen im Fettgewebe ursächlich für die Pathogenese von T2D sein. Allein durch die Bestimmung des HbA1c-Werts kann noch keine spezifischere Diagnose und daraus resultierende personalisierte Behandlung von T2D-Diabetikern erreicht werden. Eine personalisierte Behandlung lässt sich erst durch eine Unterteilung der T2D-Erkrankung in Subklassen erreichen. Bis heute gibt es kein zuverlässiges Diagnoseverfahren, das eine Subklassifizierung von Diabetikern ermöglicht. Es besteht also Bedarf an ausdifferenzierten Verfahren, sowohl in der Prognose als auch in der Diagnose von Diabetikern.

Vor diesem Hintergrund war es Aufgabe der vorliegenden Erfindung eine neue Möglichkeit anzugeben, mittels derer eine gegenüber dem Stand der Technik andere bzw. zusätzlich ausdifferenzierte Prognose und/oder Diagnose möglich ist.

Erfindungsgemäß gelöst wird diese Aufgabe durch ein Verfahren zur Prognose und/oder Diagnose einer Diabetes mellitus Typ II-Erkrankung eines Probanden, umfassend die Schritte:

a) Bestimmen des Genexpressionslevels des Genes *ADIPOQ* in einer Probe des Probanden und

b) Einordnen des Probanden unter Berücksichtigung des Genexpressionslevels des Genes *ADIPOQ* in eine von wenigstens drei Risikogruppen, wobei wenigstens zwei der möglichen Risikogruppen aus Individuen bestehen, die Diabetes mellitus Typ II bereits ausgebildet haben,

wobei zum Einordnen des Probanden in Schritt b) ferner das Genexpressionslevel des Genes HMGA2 berücksichtigt wird,

und wobei als mögliche Gruppen für eine Einordnung in Schritt b) die Gruppen

III) stark erhöhtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter ≥ 45,

IV) erhöhtes relatives Genexpressionslevel für *ADIPOQ,* erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter ≥ 45, und

V) erniedrigtes relatives Genexpressionslevel für *ADIPOQ,* erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter ≤ 45, zur Verfügung stehen.

Eine Möglichkeit eine Fehlentwicklung im Hinblick auf die Insulinresistenz und -produktion frühzeitig zu diagnostizieren liegt u.a. im Fettgewebe, das als Langzeitspeicher für Fette und Glukose eine Fehlernährung über einen sehr langen Zeitraum abpuffern kann. Vermutlich ist ein dysfunktionales Fettgewebe ein entscheidendes Element für die Entstehung von T2D, sowohl bei Übergewichtigen/Adipositas-Erkrankten als auch Normalgewichtigen. Verschiedene Studienergebnisse zeigen, dass Fettgewebe vermehrt als endokrines Organ, welches aktiv in physiologische Vorgänge eingreift bzw. diese steuert, anzusehen ist. Die vom Fettgewebe sezernierten Stoffe, Adipokine genannt, sind u.a. mit der Insulinsensitivität und -resistenz, der Fortpflanzung, der Inflammation und dem Knochenwachstum, sowie mit immunologischen Prozessen und dem Fettsäuremetabolismus assoziiert. Ein mit der Prädisposition für die Entwicklung von T2D verbundenes Adipokin ist Adiponektin (Szmitko PE, Teoh H, Stewart DJ, Verma S (2007) Adiponectin and cardiovascular disease: state of the art? Am J Physiol Heart Circ Physiol. 292:H1655-63).

Das humane Adiponektin wird vom *APM1/ACDC/ACRP30/GBP28/ADIPOQ-Gen* (Accession ID: D45371) kodiert, welches in der chromosomalen Bande 3q27 lokalisiert ist. Es enthält drei Exons die in einer 17kb großen Region liegen. Die Exons eins und zwei sind 76 bzw. 222 bp groß und sind durch Intron eins mit der Größe von 10,3 kb getrennt. Das Exon drei umfasst ungefähr 4,28 kb. Die Translation beginnt im Exon zwei und endet im Exon drei und lässt somit Exon eins und Teile des Exons drei untranslatiert. Das 30-kDa große Adiponektin-Protein wird hauptsächlich von Adipozyten produziert und sezerniert. Adiponektin besteht aus einer carboxyterminalen globulären Domäne und einer kollagenen Domäne im aminoterminalen Ende. Im Blutplasma kommt Adiponektin als vollständiges, 244 Aminosäuren langes, Protein und als proteolytisches Spaltproduktfragment, auch globuläres Adiponektin genannt, vor. Isoformen des Adiponektins entstehen aufgrund unterschiedlicher Verknüpfungen der globulären und kollagenen Domänen. Die Wirkung von Adiponektin auf die Körperzellen wird über die AdipoR1 und AdipoR2 Rezeptoren vermittelt. In erster Linie sind AdipoR1 im Skelettmuskel und AdipoR2 in Leberzellen zu finden. Weitere Studien lassen allerdings vermuten, dass AdipoR1 und AdipoR2 auch in Kardiomyozyten, Osteoblasten und β-Zellen des Pankreas exprimiert werden. Im Lipid- und Glukosemetabolismus spielt Adiponektin eine eminente Rolle. Es bewirkt eine Veränderung der Insulinsensitivität über Aktivierung der 5'Adenosinmonophosphataktivierte Proteinkinase (AMPK) und verbessert die Insulinresistenz mittels Steigerung der Fettsäureoxidation und Unterdrückung der Glukoneogenese in der Leber. Ein Mangel an Adiponektin ist neben dem erhöhten Risiko für Diabetes und einer diabetischen Angiopathie, auch mit einem erhöhten Risiko für Herzinfarkte und Schlaganfälle verbunden.

"Adiponektin" im Sinne dieses Textes ist dementsprechend das Adiponektin Protein bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. Adiponektin ist ein wichtiges Adipokin, das an der Kontrolle des Fettstoffwechsels und der Insulinsensitivität beteiligt ist und einen direkten anti-diabetischen, anti-atherogenen und entzündungshemmenden Einfluss hat. Es stimuliert die AMPK-Phosphorylierung und -Aktivierung in der Leber und dem Skelettmuskel, wodurch die Glukoseverwertung und die Fettsäureverbrennung erhöht werden. Das humane Adiponektin-Gen ist in der chromosomalen Region 3q27 lokalisiert und besteht aus drei Exons, die sich über einen ≥17kb langen Bereich erstrecken. Es codiert u.a. ein 244 Aminosäuren langes vollständiges Adiponektin-Protein, dessen molekulare Masse 30 kDa beträgt. Das Adiponektin-Protein ist durch eine carboxyterminalen globulären Domäne und einer kollagenen Domäne im aminoterminalen Ende gekennzeichnet. Grundsätzlich kommt Adiponektin im Plasma als vollständiges Protein (244 Aminosäuren) sowie als proteolytisches Spaltproduktfragment, auch globuläres Adiponektin genannt, vor. Die Isoformen des Adiponektins entstehen aufgrund unterschiedlicher Verknüpfungen der globulären und kollagenen Domänen. Im Plasma zirkulieren vor allem drei Hauptkomplexe, ein niedermolekulares Trimer (LMW), ein mittelmolekulares Hexamer (MMW) und ein hochmolekularer Komplex (HMW).

Der Wert für das relative Genexpressionslevel im Sinne der vorliegenden Erfindung kann auf jede dem Fachmann bekannte Weise erfolgen. Bevorzugt ist eine Bestimmung des Genexpressionslevels auf mRNA-Ebene oder auf Proteinebene. Bevorzugt ist dabei die mRNA-Ebene.

Bei der vorliegenden Erfindung werden relative Genexpressionslevel bestimmt. Sofern nachfolgend lediglich von "Genexpressionslevel" gesprochen wird, handelt es sich - sofern nicht anders angemerkt - stets um relative Genexpressionslevel. Die relativen Genexpressionslevel werden dabei bevorzugt durch Bestimmung des Genexpressionslevels des zu untersuchenden Gens im Verhältnis zum Expressionslevels eines housekeeping Gens, bevorzugt ausgewählt aus der Gruppe bestehend aus *HPRT,* 18S rRNA, *GAPDH, GUSB, PBGD, B2M, ABL, RPLP0,* wobei ganz besonders *HPRT* bevorzugt ist, bestimmt.

Der Begriff "Prognose" bedeutet im Sinne dieses Textes eine Vorhersage eine erhöhte Wahrscheinlichkeit der Entwicklung oder des Auftretens eines klinischen Zustandes oder einer Krankheit.

Der Begriff "Diagnose" einer Krankheit bedeutet im Sinne dieses Textes, dass eine Krankheit, die bereits klinische Symptome zeigt, identifiziert und/oder bestätigt wird.

Diabetes mellitus Typ II wird nachfolgend im Text auch mit T2D abgekürzt.

"Proband" im Sinne der vorliegenden Anmeldung sind Mensch und Tier, wobei bevorzugt Menschen gemeint sind.

"Risikogruppen" im Sinne dieses Textes sind solche Gruppen, die durch geeignete Unterscheidungsmerkmale voneinander getrennt werden können und jeweils ein gemeinsames erhöhtes oder nicht erhöhtes Risiko in Bezug auf die Entwicklung oder das Vorhandensein einer Krankheit insbesondere T2D besitzen. Zudem können sich Risikogruppen zusätzlich durch weitere physiologische Unterschiede voneinander unterscheiden, was ggf. therapeutische oder prophylaktische Relevanz besitzt.

Überraschenderweise hat sich herausgestellt, dass es aufgrund des erfindungsgemäßen Verfahrens möglich ist, innerhalb der Gruppe der Diabetes mellitus Typ II erkrankten Individuen (Probanden) eine weitere Unterscheidung vorzunehmen. Diese Unterscheidung hilft beim Auffinden geeigneter Therapien für das jeweilige Individuum.

Ein solcher Therapieansatz könnte beispielsweise sein, dass in Abhängigkeit von der ADIPOQ-Expression innerhalb der T2D-Erkrankten Medikamente gegeben werden, die den Adiponektin-Spiegel beeinflussen. So könnte es beispielsweise möglich sein, bei den Gruppen relativ geringer *ADIPOQ*-Expression den Adiponektin-Spiegel anzuheben. Ein geeignetes Medikament hierfür könnte Metformin sein.

Vor dem erfindungsgemäßen Verfahren wird natürlich eine entsprechende Probe eines Probanden bereitgestellt.

Wie bereits bei beschrieben, ergibt sich überraschenderweise durch das Einbeziehen des relativen Genexpressionslevels des Genes *ADIPOQ* in einer von wenigstens drei Risikogruppen die Möglichkeit, innerhalb der bereits erkrankten Diabetes mellitus Typ II-Probanden eine weitere Differenzierung festzustellen, die wiederum für therapeutische Zwecke genutzt werden kann.

Erfindungsgemäß ist es in der vorliegenden Erfindung, dass beim Einordnen der Probanden in Schritt b) ferner das Genexpressionslevel des Genes *HMGA2* (und ggf. IL-6) berücksichtigt wird.

"HMGA2" im Sinne dieses Textes ist das High Mobility Group AT-Hook Protein 2 (HMGA2) bzw. dessen Gen bzw. die zugehörige mRNA und/oder Teile dieses Proteins bzw. Gens (bzw. dessen mRNA) bevorzugt wenigstens eine Aminosäurenkette $\geq$ 7 Aminosäuren, weiter bevorzugt $\geq$ 15 Aminosäuren und besonders bevorzugt $\geq$ 20 Aminosäuren bzw. eine Nucleinsäurenkette von $\geq$ 20 Nucleinsäuren, weiter bevorzugt $\geq$ 40 Nucleinsäuren und besonders bevorzugt $\geq$ 55 Nucleinsäuren ggf. pro Strang. HMGA2 ist ein Transkriptionsfaktor der die Regulation der Genexpression beeinflusst und zur Gruppe der High Mobility Group A-Proteine (HMGA-Proteine) gehört. Die HMGA-Proteine sind Chromatin-assoziierte, säurelösliche Nicht-Histon-Proteine, die an sequenzunabhängige, spezifische Motive der DNA binden. Als architektonische Transkriptionsfaktoren erhöhen bzw. inhibieren sie über strukturelle Änderungen der Chromatinorganisation die Bindungsfähigkeit weiterer Transkriptionsfaktoren. Das humane *HMGA2*-Gen ist in der chromosomalen Region 12q14~15 lokalisiert und besteht aus fünf Exons, die sich über einen $\geq$160kb langen Bereich erstrecken. Es codiert ein 109 Aminosäuren langes Protein, dessen molekulare Masse 12 kDa beträgt. Das HMGA2-Protein ist durch drei stark konservierte DNA-bindende Domänen, die sogenannten AT-Hooks und eine saure negativ geladene C-terminale Domäne gekennzeichnet.

"PPAR-gamma" im Sinne dieses Textes ist der Peroxisom-Proliferator-aktivierte Rezeptor gamma (PPAR-gamma) bzw. dessen Gen bzw. die zugehörige mRNA und/oder Teile dieses Proteins bzw. Gens (bzw. dessen mRNA) bevorzugt wenigstens eine Aminosäurenkette $\geq$ 7 Aminosäuren, weiter bevorzugt $\geq$ 15 Aminosäuren und besonders bevorzugt $\geq$ 20 Aminosäuren bzw. eine Nucleinsäurenkette von $\geq$ 20 Nucleinsäuren, weiter bevorzugt $\geq$ 40 Nucleinsäuren und besonders bevorzugt $\geq$ 55 Nucleinsäuren ggf. pro Strang. PPAR-gamma ist ein Liganden-bindender nukleärer Transkriptionsfaktor der PPAR-Unterfamilie, der zur Gruppe der nukleären Hormonrezeptoren gehört. Über Heterodimerisation mit dem Retinoid X Rezeptor $\alpha$ (RXR$\alpha$) aktiviert PPAR-gamma die Transkription verschiedener Gene. Das humane *PPAR-gamma*-Gen ist in der chromosomalen Bande 3p25 lokalisiert und besteht aus 11 Exons. Das humane *PPAR-gamma*-Gen codiert für 2 Isoformen, die jeweils ein 477 und ein 505 Aminosäuren langes Protein darstellen.

"IL-6" im Sinne dieses Textes ist das Interleukin-6 bzw. dessen Gen bzw. die zugehörige mRNA und/oder Teile dieses Proteins bzw. Gens (bzw. dessen mRNA) bevorzugt wenigstens eine Aminosäurenkette ≥ 7 Aminosäuren, weiter bevorzugt ≥ 15 Aminosäuren und besonders bevorzugt ≥ 20 Aminosäuren bzw. eine Nucleinsäurenkette von ≥ 20 Nucleinsäuren, weiter bevorzugt ≥ 40 Nucleinsäuren und besonders bevorzugt ≥ 55 Nucleinsäuren ggf. pro Strang. Interleukin-6 ist ein Zytokin, das sowohl in Entzündungsreaktionen als auch bei der Reifung von B-Lymphozyten eine Rolle spielt. Darüber hinaus wurde nachgewiesen, dass es sich um einen endogenen, entzündlich wirkenden Stoff, ein sogenanntes Pyrogen handelt, das beim Vorliegen von Autoimmunerkrankungen oder Infektionen hohes Fieber auslösen kann. Das Protein wird vorwiegend an Orten akuter oder chronischer Inflammation erzeugt, von wo aus es ins Serum sezerniert wird und über den Interleukin-6-Rezeptor alpha eine inflammatorische Reaktion auslöst. Interleukin-6 ist in verschiedene entzündungsassoziierte Krankheitszustände involviert, darunter eine Prädisposition für Diabetes mellitus oder die systemische juvenile idiopathische Arthritis (Still-Syndrom). Das humane *IL-6*-Gen ist in der chromosomalen Bande 7p15.3 lokalisiert und besteht aus sechs Exons. Das IL-6-Präkursorprotein besteht aus 212 Aminosäuren. Nach Abspaltung eines 28 Aminosäuren langen Signalpeptids besitzt das reife Interleukin-6 eine Länge von 184 Aminosäuren (Hirano T, Yasukawa K, Harada H, Taga T, Watanabe Y, Matsuda T, Kashiwamura S, Nakajima K, Koyama K, Iwamatsu A, et al., 1986. Complementary DNA for a novel human interleukin (BSF-2) that induces B lymphocytes to produce immunoglobulin. Nature 324: 73-76).

Es hat sich dabei herausgestellt, dass bei der erfindungsgemäßen Verwendung bzw. bei dem erfindungsgemäßen Verfahren die Kombination der Daten für *ADIPOQ* mit denen der genannten bevorzugten Gene zu besonders sicheren Gruppenunterscheidungen innerhalb der Risikogruppen führen.

Bevorzugt ist ein erfindungsgemäßes Verfahren, wobei für die Eingruppierung in Risikogruppen (in Schritt c)) ferner eines oder mehrere Merkmale des Probanden ausgewählt aus der Gruppe bestehend aus Alter, BMI, Größe, Gewicht, Geschlecht, Bauch- & Hüftumfang, Körperfettanteil, Muskelmasse, Total Body Water (TBW), Blutdruck, Raucherstatus, Bluthochdruck, Einnahme blutdrucksenkender Medikamente berücksichtigt wird.

Somit ist es möglich, mittels der Berücksichtigung weiterer Merkmale der Probanden eine zusätzliche Sicherheit der Aufspaltung innerhalb der Risikogruppen zu erzeugen. Teilweise ist es selbstverständlich auch möglich, mit jedem zusätzlichen Marker (der Berücksichtigung eines weiteren Merkmals) die Aufspaltung der Risikogruppen noch weiter zu verfeinern.

Es ist bekannt, dass mittels der Blutwerte relevante Aussagen über den Status hinsichtlich von T2D gemacht werden können.

Mithilfe des erfindungsgemäßen Einsatzes von *ADIPOQ* lassen sich in Kombination mit den zusätzlichen Markern/Blutparametern weitere Gruppenausdifferenzierungen erzeugen.

Die nachfolgende Tabelle gibt Hinweise zu typischen Werten für Blutparameter mit einem Normreferenzbereich laut Leitlinie der Deutschen Diabetes Gesellschaft von 2012 sowie der ESC/EAS Guidelines für the Management of Dyslipidaemias von 2016:

**Tabelle 1**

| Parameter | Norm-Referenzbereich (mg/dL) | In T2D-Gruppe | In Nicht-T2D-Gruppe |
|---|---|---|---|
| Triglyceride | < 150 | Erhöht | Nicht-erhöht /niedrig |
| Cholesterin, ges* | **<20 Jahre: <170**<br><br>**20-30 Jahre: <200**<br>**30-40 Jahre: <220**<br>**>40 Jahre: <240** | Erhöht | Nicht-erhöht /niedrig |
| HDL-Cholesterin | Männer > 40<br>Frauen > 48 | Erniedrigt | Erhöht |
| non- HDL-Cholesterin + | **-Sehr hohes Risiko:** < 100<br><br>**-hohes Risiko:** <130<br>**-Niedriges bis moderates Risiko:** <145 | Erhöht | Nicht-erhöht /niedrig |

(fortgesetzt)

| Parameter | Norm-Referenzbereich (mg/dL) | In T2D-Gruppe | In Nicht-T2D-Gruppe |
|---|---|---|---|
| LDL-Cholesterin + | **-Sehr hohes Risiko: <70**<br><br>**-hohes Risiko: <100**<br>**-Niedriges bis moderates Risiko: <115** | Erhöht | Nicht-erhöht /niedrig |
| CRP | < 5,0 mg/L | Erhöht | Nicht-erhöht /niedrig |
| Nüchtern-Glukose (venöses Plasma) | < 100 | Erhöht | Nicht-erhöht /niedrig |
| 2h-Glukosewert (venöses Plasma) | <120 | Erhöht | Nicht-erhöht /niedrig |
| Präprandiale Glukose (venöses Plasma) | < 100 | Erhöht | Nicht-erhöht /niedrig |
| HbA1c | < 5,7% | Erhöht | Nicht-erhöht /niedrig |

[0031]    * Der durchschnittliche Gesamtcholesterinspiegel der Altersgruppe zwischen 35 und 65 Jahren in Deutschland liegt bei etwa 236 mg/dl, die Standardabweichung bei ±46 mg/dl.

+ Die Abstufung basiert auf sehr hohem, hohem und niedrigen bis moderatem Risiko für kardiovaskuläre Erkrankungen (s. aktuelle Leitlinien von European Society of Cardiology (ESC) and European Atherosclerosis Society (EAS) für 2016: ESC/EAS Guidelines for the Management of Dyslipidaemias). Das Risiko kann mit einem SCORE (Systematic Coronary Risk Estimation) errechnet werden. Die verbreitesten Score-Systeme sind der ESC-SCORE und der PROCAM-SCORE (Prospective Cardiovascular Munster Study).

Hierbei kann bei der Differenzierung des Status der jeweiligen Blutwerte erfindungsgemäß eine gute Kombination mit anderen Markern zur zusätzlichen Aufspaltung innerhalb der Risikogruppen bei geeigneter Auswertung (vgl. auch unten) führen.

Bevorzugt ist ein erfindungsgemäßes Verfahren, wobei die Probe aus Fettgewebe gewonnen wurde.

Dabei ist bei der "Probe aus Fettgewebe" diejenige Probe zu verstehen, die die Werte für wenigstens ein Genexpressionslevel, bevorzugt für alle Genexpressionslevel liefert, die für Schritt b) verwendet werden. Selbstverständlich können die Blutwerte nicht aus Fettgewebe gewonnen werden.

Bevorzugt ist dabei für das bevorzugte erfindungsgemäße Verfahren, dass die Probe aus Fettgewebe durch Punktion des subkutanen abdominalen Fettgewebes gewonnen wurde.

Durch fächerförmige Punktierungen unter Sog lassen sich besonders gut Zellen und Zellverbände gewinnen, die eine molekulargenetische Analyse ermöglichen. Das fächerförmige Vorgehen bei der Punktion vermindert zum einen eine Verklebung/Verstopfung der Kanülenspitze mit Fettzellen, zum anderen erhält man Zellen aus verschiedenen Regionen des betreffenden Fettgewebes und hat somit einen repräsentativen Querschnitt der Verteilung unterschiedlicher Fettgewebszelltypen.

Besonders bevorzugt ist ein erfindungsgemäßes Verfahren, wobei die Probenmasse für die Proben aus Fettgewebe ≤ 50 mg, bevorzugt ≤ 20 mg und weiter bevorzugt ≤ 5 mg ist.

Überraschenderweise hat sich gezeigt, dass selbst bei sehr kleinen Probevolumen aus Fettgewebe zuverlässig differenzierte Ergebnisse erzielt werden können. Dabei ist es besonders bevorzugt, dass die Probe durch Punktion als Feinnadelaspirat gewonnen wurde.

Die Bestimmung der jeweiligen Parameter aus Fettgewebe besitzt aus Sicht der Erfinder auch im Sinne der Prognose folgende Vorteile: Zum einen können nach der Bestimmung der Parameter aus Fettgewebe erfindungsgemäß unterschiedliche Risikogruppen von Individuen, die bereits an Diabetes mellitus Typ II erkrankt sind, erkannt werden ohne dass z.B. weitere Bestimmungen von Blutproben erforderlich sind. Zum anderen ermöglicht die erfindungsgemäße Bestimmung der Parameter aus Fettgewebe frühzeitiger Individuen zu identifizieren, die eine erhöhte Wahrscheinlichkeit der Ausbildung eines Diabetes mellitus Typ II besitzen als z.B. herkömmliche HbA1c-Tests. Diese HbA1c-Tests geben nur Auskunft über den Blutglukose-Spiegel der letzten vier bis 12 Wochen wieder und sind somit für eine längerfristige Prognose zur Ausbildung eines Diabetes mellitus Typ II eher weniger geeignet.

Erfindungsgemäß bevorzugt ist der Proband ein Mensch, da eine differenzierte Prognose und Diagnose im Falle von T2D bei Menschen von einer ganz besonderen Bedeutung sowohl wirtschaftlicher als auch gesundheitspolitischer Natur ist.

Wie bereits oben angedeutet ist es bevorzugt, dass die Bestimmung des Genexpressionslevels auf mRNA-Ebene erfolgt. So können zuverlässige Daten mit äußerst geringen Probemengen und mittels etablierter Methoden gewonnen werden.

Bevorzugt ist ein erfindungsgemäßes Verfahren, wobei die Eingruppierung in die Risikogruppen (in Schritt b)) unter Verwendung des multivariaten Modells der selbstorganisierenden Karten nach Kohonen erfolgt.

Zu der Methodik der selbstorganisierenden Karten wird auf den untenstehenden Methodikteil verwiesen.

Erfindungsgemäß bevorzugt ist ein Verfahren, wobei die Einordnung in Schritt b) in eine von wenigstes 5 Gruppen erfolgt, wobei wenigstens zwei der Gruppen aus Individuen bestehen, die eine erhöhte Wahrscheinlichkeit der Ausbildung eines Diabetes mellitus Typ II besitzen.

Es hat sich nämlich herausgestellt, dass mittels der Berücksichtigung des relativen Genexpressionslevels von *ADIPOQ* (bzw. alternativ aber nicht erfindungsgemäß damit linear statistisch korrelierter Gene) es möglich ist, auch innerhalb der Gruppen der Individuen/Probanden, die eine erhöhte Wahrscheinlichkeit zur Ausbildung eines Diabetes mellitus Typ II besitzen, eine Differenzierung auszubilden. Auch aus dieser Erkenntnis lassen sich therapeutische Ansätze ableiten bzw. geeignete präventive Maßnahmen.

Weiter bevorzugt ist ein erfindungsgemäßes Verfahren, wobei das Genexpressionslevel auf mRNA-Ebene relativ zu dem Genexpressionslevel eines housekeeping Gens gemessen wird. Diese Methodik eignet sich in besonderem Maße dazu, zuverlässige Ergebnisse aus geringen Probenmengen zu erhalten.

Bevorzugt ist ein erfindungsgemäßes Verfahren, wobei als mögliche Gruppen für eine Einordnung in Schritt b) die Gruppen mit der Markersituation

I) erniedrigtes relatives Genexpressionslevel für *ADIPOQ*, stark erhöhtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 45, und

II) erniedrigtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 45, zur Verfügung stehen.

In diesen beiden bevorzugten zur Verfügung stehenden Eingruppierungsmöglichkeiten drückt sich das zusätzliche Potential der Verwendung der Daten von *ADIPOQ* in besonderer Form aus. Mit den jeweiligen Kombinationen der Markerwerte (die Kombination von stets drei Werten) lassen sich - neben den Eingruppierungsmöglichkeiten in weitere Gruppen -die erfindungsgemäß bevorzugten Eingruppierungsmöglichkeiten anwenden. Hierdurch ist es möglich, eine zuverlässige zusätzliche Aussage einer Unterscheidbarkeit der mit T2D erkrankten Probanden/Individuen zu gewinnen.

Im erfindungsgemäßen Verfahren, gibt es als mögliche Gruppen für die Einordnung in Schritt b) (zumindest) die Gruppen mit der Markersituation

III) stark erhöhtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 45,

IV) erhöhtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\geq$ 45, und

V) erniedrigtes nicht erhöhtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\leq$ 45, zur Verfügung stehen.

In den Gruppen IV und V sind nur Individuen enthalten, die die Krankheit nicht ausgebildet haben. Die Gruppe III umfasst Individuen, die bereits klinische Symptome eines T2D zeigen.

Hinsichtlich der Beschreibung "erhöht"/"erniedrigt" gilt im Zweifelsfall folgendes: Für die relativen Genexpressionslevel liegt ausgehend vom Mittelwert des jeweiligen Patientenkollektives folgende Situation vor:

| +/- 5% | unverändert |
| + 5-10 % | leicht erhöht |
| - 5-10 % | leicht erniedrigt |
| + 5-50% | erhöht |
| - 5-50 % | erniedrigt |
| +/ > 50 % | stark erhöht |
| -/ > 50 % | stark erniedrigt |

Für die Blutwerte gilt die Abstufung entsprechend, wobei hier als Bezugswert der in Tabelle 1 angegebene Wert gilt und nur der Bereich betrachtet wird, der jenseits des Normenbereichs liegt (es gibt also stets entweder nur "erhöhte" oder "erniedrigte" Werte der jeweiligen Abstufungen neben den "unveränderten" Werten.

Selbstverständlich umfasst das Patenten kollektiv, das Grundlage ist, stets auch bereits an T2D erkrankte Individuen.

Zur Aufteilung der Gruppen wird auch auf die Beispiele verwiesen.

Von Interesse ist auch ein Kit für ein erfindungsgemäßes Verfahren, umfassend

a) ein Primerpaar, das an die cDNA von *HMGA2* bindet und

b) ein Primerpaar, das an die cDNA von *ADIPOQ* bindet und bevorzugt

c) ein Primerpaar, das an die cDNA eines housekeeping Gens ausgewählt aus der Gruppe bestehend aus *HPRT, 18S rRNA, GAPDH, GUSB, PBGD, B2M, ABL, RPLP0 bindet.*

Mit diesem Kit ist es möglich, für das erfindungsgemäße Verfahren die Genexpressionslevel von *ADIPOQ* und *HMGA2* festzustellen. Die genannte Primerpaarkombination eignet sich somit für eine bevorzugte Variante des erfindungsgemäßen Verfahrens.

Nachfolgend wird die Erfindung anhand von Beispielen und unter Berücksichtigung der jeweiligen Methodik näher erläutert.

Auswerteverfahren

Methodik

Ziel der angewendeten Methoden war es, neue Klassifikationen (Cluster) von Diabetikern und Nichtdiabetikern auf der Basis verschiedener Biomarker wie z. B. HMGA2, ADIPOQ, IL-6 oder PPAR-gamma zu erstellen, die diagnostisch aber auch zukünftig therapeutisch die bisherigen Klassifikationen erweitern.

Zur formalen Beschreibung der Studiendaten wurden die üblichen Methoden der deskriptiven Statistik verwendet. Für nominale Parameter wurden absolute und relative Häufigkeit angegeben, für ordinale Parameter zusätzlich der Median. Für metrische Werte wurden Mittelwert und Standardabweichung berechnet. Normalverteilungen wurden mit Hilfe des Kolmogorow-Smirnow-Tests (KS-Test) überprüft. Nicht-parametrische Korrelationen zwischen den Biomarken wurden mit Hilfe von Kendall tau-b berechnet. Für Vergleiche zwischen kategoriellen Variablen wurde der $\chi 2$ Test verwendet:

Zur Berechnung der a priori nicht bekannten Cluster wurden selbstorganisierende Karten (selforganizing maps (**SOM**)) verwendet. SOMs (hier Kohonen-Karten nach Teuvo Kohonen, vgl. Teuvo Kohonen: Self-Organizing Maps. Springer-Verlag, Berlin 1995, ISBN 3-540-58600-8) sind Typen von künstlichen neuronalen Netzen mit einem unüberwachten Lernverfahren mit dem Ziel eine topographische Merkmalskarte in Form von Clustern des Eingaberaums (Patientendaten) zu erreichen. Patienten innerhalb eines Clusters sollen hierbei maximal homogen und zwischen den Clustern maximal inhomogen sein. SOMs werden verwendet zum Clustering, zur Visualisierung komplexer Zusammenhänge, Vorhersage (Auswertung), Modellierung und Datenexploration. Das hier verwendete Netz besteht aus 1000 Neuronen, Korrelationen wurden automatisch kompensiert und fehlende Werte berücksichtigt. Zur Erzeugung der Cluster wurde das SOM-WARD clustering-Verfahren verwendet (2-stufiger hierarchischer Clusteralgorithmus). Farbkodierungen wurden mit Heat Maps durchgeführt. Die hierdurch erzeugten Cluster wurden deskriptiv verglichen. Zur Beschreibung der Cluster mit Hilfe von Entscheidungsbäumen bzw. Fakten und Regeln wurden verschiedene Klassifikationsalgorithmen verwendet wie C5.0, CART und Exhausted Chaid. Als Gütemaß für die verschiedenen Klassifikatoren wurden Klassifikationsgenauigkeit, Kompaktheit des Modells, (z.B. Größe eines Entscheidungsbaums) Interpretierbarkeit des Modells, Effizienz und Robustheit gegenüber Rauschen und fehlenden Werten bewertet.

Zur weiteren Validierung der Modelle und zur Berechnung der Wichtigkeit der Biomarker für die verschiedenen Klassifikationsmodelle wurden als Vorhersagemodell RBF-Netze (Radiale Basisfunktionen Netzwerke) erstellt. Durch die RBF-Netze erhält man eine geeignete Approximation der Clusterzuordnung der SOMs. Die Eingabevektoren wurden normalisiert (Subtraktion des Mittelwerts und Division durch den Bereich (x-Min)/(Max-Min); Normalisierte Werte liegen im Bereich zwischen 0 und 1). Als Aktivierungsfunktion wird die Softmax-Funktion $\sigma$ als normalisierte radiale Basisfunktion verwendet. Softmax $\sigma$ bildet einen k-dimensionalen Vektor z auf einen k-dimensionalen Vektor $\sigma(z)$ ab.

Die Netzleistung (wie "gut" ist das Netz) wurde anhand folgender Daten überprüft:

- *Modellzusammenfassung:* Ergebnisse einschließlich Fehler, Relativer Fehler oder Prozentsatz der falschen Vorhersagen.

- *Klassifikationsergebnisse:* Für jede abhängige Variable wurde eine Klassifikationstabelle angegeben.

- *ROC-Kurven:.* ROC-Kurven (Receiver Operating Characteristic curves) geben die Sensitivität und Spezifität für jeden möglichen Cutpoint der Eingangsvariablen an. Die Area under the Curve AUC ist ein Maß für die Qualität der Klassifikation. sowie

- *Kumulative Gewinndiagramme.*

Es wurden im Einzelnen folgende Methoden verwendet:

1. Selbstorganisierende neuronale Netzwerke (-7 Kohonen-Karten)

2. Klassifikationsalgorithmen

- Entropiebasierte Lernverfahren (C5.0)

- Exhausted Chaid und

- CART

3. Radiale Basisfunktionen (spezieller Typ von neuronalen Netzen)

4. Deskriptive und induktive Statistik

**1) Selbstorganisierende neuronale Netzwerke**

Als Selbstorganisierte Karten (selforganizing maps (**SOM**)) bezeichnet man Typen von künstlichen neuronalen Netzen mit einem unüberwachten Lernverfahren mit dem Ziel eine topologische Darstellung des Eingaberaums (hier Patientendaten) zu erreichen. Die bekanntesten SOMs sind die topologieerhaltenden Kohonen-Karten nach Teuvo Kohonen. Der Lernalgorithmus erzeugt selbständig Klassifikatoren, nach denen es die Eingabemuster in (bisher nicht bekannte) Cluster einteilt. Ziel ist es, zu erreichen, dass die Patienten innerhalb eines Clusters maximal homogen und zwischen den Clustern maximal inhomogen sind.

**Kernidee** (Topographische Merkmalskarte): "Benachbarte" Eingabevektoren (hier Patientendaten) sollten zu benachbarten Neuronen in der Karte gehören so dass die Dichte und Verteilung der Neuronen dem Wahrscheinlichkeitsmodel der Trainingsmenge entsprechen.

**Vorteile:** Nachbarschaftsbeziehungen im "unübersichtlichen" Inputraum können direkt in der Ausgabeschicht abgelesen werden.

**Anwendungen:** SOMs werden verwendet zum Clustering, zur Visualisierung komplexer Zusammenhänge, Vorhersage (Auswertung), Modellierung und Datenexploration. Die Schwerpunkte der Anwendung für die hier vorliegenden Fragestellungen sind Clustering, Visualisierung und Vorhersage.

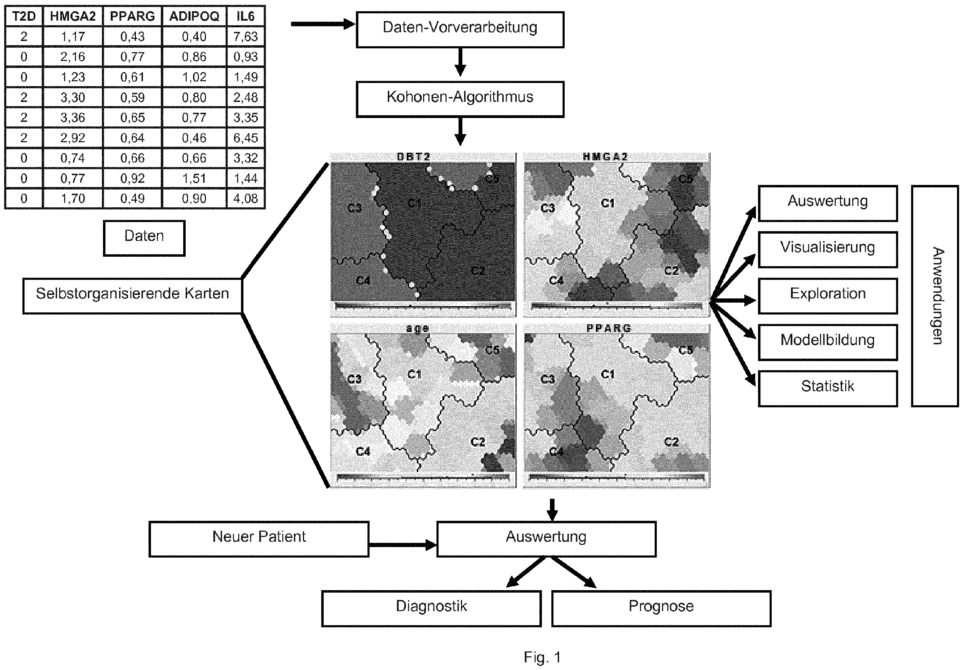

(Werkzeuge: z.B. **Self Organizing Maps in R** (R ist eine freie Programmiersprache für statistische Berechnungen und Grafiken. R ist Teil des GNU-Projekts, vgl. auch https://cran.r-project.org/web/packages/som/som.pdf und Figur 1)

### 1.1) Formale Beschreibung des Kohonen-Netzwerkmodells (Algorithmus)

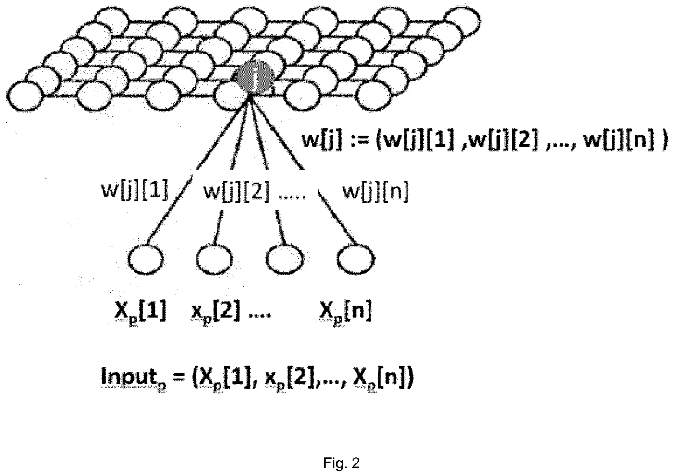

- Ein SOM besteht aus zwei Schichten von Neuronen **(Eingabeschicht** und **Ausgabeschicht),** vgl. auch Figur 2.

- Jedes Neuron der Eingabeschicht ist mit jedem Neuron der Ausgabeschicht verbunden (die Neuronen der Eingabeschicht sind vollständig mit denen der Ausgabeschicht vernetzt). Jedes Neuron der Eingabeschicht entspricht einem Parameter des Datensatzes. Die Anzahl der Eingabeneuronen ist die Dimension der Eingabeschicht. Die Ausgabeneuronen stehen durch eine Nachbarschaftsfunktion miteinander in Beziehung.

- Die Stärke der Verbindung wird durch eine Zahl **(=Gewicht) w[i][j]** repräsentiert (w[i][j] ist das Gewicht, das die Stärke der Verbindung zwischen dem i-ten Eingabeneuron und dem j-ten Ausgabeneuron angibt). Der Vektor w[j] repräsentiert alle Gewichte w[i][j] (i=1 ...n, n ist die Anzahl der für jeden Patienten erfassten Parameter) zu dem j-ten Ausgabeneuron. Eingabevektoren und Gewichtsvektoren sind normalisiert (Länge=1).

- **Initialisiere die Gewichtsvektoren.** Als Startvorgabe werden für die Gewichte beliebige durch einen Zufallsgenerator erzeugte Werte vorgegeben.

- Jeder Patient p definiert durch seine Werte einen Eingabevektor **Input$_p$ = ($x_p$[1], $x_p$[2],..., X$_p$[n])** mit den Komponenten Input$_p$[i] = $x_p$[i]. Diese Eingabevektoren werden zuerst auf 1 normalisiert.

- Für jeden Patienten p und jedes Neuron j in der Ausgabeschicht wird der euklidische Abstand

$$s_p[j] = \| \mathbf{w}[j] - \mathbf{input}_p \| = \sqrt{\sum_i \left( \mathbf{w}[j][i] - \mathbf{input}_p[i] \right)^2}$$

zwischen dem Gewichtsvektor w[j] und dem Eingabevektor Input$_p$ berechnet.

- Das Ausgabeneuron das den kleinsten Abstand zum Eingabevektor Input$_p$ hat, heißt **Gewinnerneuron ("Winner-Takes All").** Der Gewichtsvektor des Gewinnerneurons ist dem Eingabevektor am ähnlichsten d.h. hat die "maximale Erregung" unter dem Eingabevektor Input$_p$. Sollten 2 oder mehr Ausgabeneuronen den gleichen minimalen Abstand haben, so wird per Zufallsgenerator ein Neuron ausgewählt.

- Das **Gewinnerneuron** sowie seine Nachbarn erhalten den "Zuschlag", dürfen also den Input repräsentieren

- Hiermit ist eine Funktion f(input$_p$) definiert, die jedem Vektor input$_p$ des Inputraumes (Musterraum, Merkmalsraum) einen Ort $\alpha$ in einer repräsentierenden Schicht (Karte) zuweist
f: input$_p$ →f(input$_p$) .= arg min(llw[j] - input$_p$ll). Das Minimum wird über alle Gewichtsvektoren w[j] gebildet. Die Funktion arg liefert den Index des Gewinnerneurons.

- Bestimme für jeden Patienten (Eingabemuster) das Gewinnerneuron nach obiger Vorschrift.

- **Nachbarschaftsfunktion** und **Gewichtsadaption:** Im nächsten Schritt werden die Gewichte des Siegerneurons und die seiner umliegenden Neuronen adaptiert. Dabei spielt der Grad der Nachbarschaft zum Gewinnerneuron eine große Rolle. Nehmen wir an, das Gewinnerneuron hat den Index $\alpha$. Für einen Input[i] und ein Ausgabeneuron j bezeichne Dw[j][i] die Gewichtsänderung im Rahmen einer Lernregel. Diese wird wie folgt berechnet:

$$\mathbf{Dw[j][i] = \eta(t) * (input[i] - w[i][j]) * NbdWt(\alpha, j)}$$

wobei

$\eta$**(t) die Lernrate** ($\eta$(t) mit 0 < $\eta$(t) < 1) und
NbdWt($\alpha$, j) die **Nachbarschaftsfunktion** (neighborhood weighting Funktion) bezeichnet. Eine Nachbarschaftsfunktion berechnet den Grad der Nachbarschaft eines Ausgabeneurons zum Gewinnerneuron, welcher zwischen 0 und 1 liegt. Konvergenzbeweise gegen einen statistisch beschreibbaren Gleichgewichtszustand existieren z.B. für $\eta$**(t) := $\eta$\*t$^{-a}$ mit 1<a$\leq$1.**

- **Nähere Gewichtsvektoren proportional zur Nachbarschaftsfunktion zueinander an** Die Gewichtsvektoren w[j] aller Neuronen j gemäß der Nachbarschaftsfunktion NbdWt(a, j) des Neurons $\alpha$ werden aktualisiert. Das neue Gewicht **w$_{neu}$[i][j]** berechnet sich wie folgt:

- **w$_{neu}$[j][i] := w[j][i] + Dw[j][i]**

- Die Nachbarschaftsfunktion kann z.B. eine der folgenden Funktionen sein:

$$NbdWt(\alpha, j) = \frac{1}{1 + \left(\dfrac{d(\alpha, j)}{s}\right)^2}$$

$$NbdWt(\alpha, j) = e^{-\left(\dfrac{d(\alpha, j)}{s}\right)^2}$$

mit d($\alpha$, j):= ( $\alpha$ - j); s ist ein Skalar. Die Breite s = s(t) der Nachbarschaftsfunktion und die Lernschrittweite $\eta$(t) werden im Lauf der Zeit verringert: Das GewinnerNeuron und seine Nachbarn werden gemäß der Funktion NbdWt(a, j) "aktiviert".

- Normiere Gewichtsvektoren auf Länge 1, präsentiere nächsten Datenpunkt.
Für die verschiedenen Verfahren zur Farbkodierungen (Heat Maps) von Karte wird auf die Literatur verwiesen (z.B. http://citeseerx.ist.psu.edu/viewdoc/download?doi=10.1.1.100.500&rep=rep1&type =pdf, https://arxiv.org/pdf/1306.3860.pdf oder https://www.visualcinnamon.com/2013/07/self-organizing-maps-creating-hexagonal.html ).

**2) Klassifikationsalgorithmen**

Zur Auswertung der SOM-Modelle (Beschreibung der Klassen durch Fakten und Regeln bzw. Entscheidungsbäume) werden 3 verschiedene Klassifikationsalgorithmen verwendet: (1) Entropiebasierte Lernverfahren (C5.0), (2) Exhausted Chaid und (3) CART.

**2.1) Definition.**

Klassifikationsverfahren sind Methoden und Kriterien zur Klassifizierung von Objekten (hier Patienten) in Klassen (hier Typen und Subtypen von gesunden Prädiabetikern bzw. Diabetikern).
Aus einer Trainingsmenge von Beispielen mit bekannter Klassenzugehörigkeit wird mit Hilfe des Klassifikationsalgorithmus ein **Klassifikator in Form von Entscheidungsbäumen oder äquivalent in Form von Fakten und "Wenn-Dann" Regeln** generiert. Die Klassifikation grenzt sich vom Clustering (s.a. SOMs) dadurch ab, dass bei der **Klassifikation** die Klassen apriori bekannt sind, während beim **Clustering** die Klassen erst gesucht werden müssen.
**Entscheidungsbäume** dienen zur Entscheidungsfindung durch eine baumartige Struktur, die aus einem Wurzelknoten (Startknoten), Knoten, Kanten und Blättern (Endknoten) bestehen (Figur 2).
Formal ist ein Baum ein endlicher Graph mit den Eigenschaften:

1) Es gibt genau einen Knoten, in dem keine Kante endet (die **"Wurzel").**

2) In jedem von der Wurzel verschiedenen Knoten endet genau eine Kante, die Kanten sind gerichtet

3) Jeder Knoten ist von der Wurzel auf genau einem Pfad erreichbar.

Ein **Entscheidungsbaum** ist ein Baum:

1) Jeder Knoten testet ein Attribut

2) Jeder Zweig korrespondiert zu einem Attributwert

3) Jedes Blatt (Knoten ohne ausgehende Kanten) ordnet eine Klasse zu.

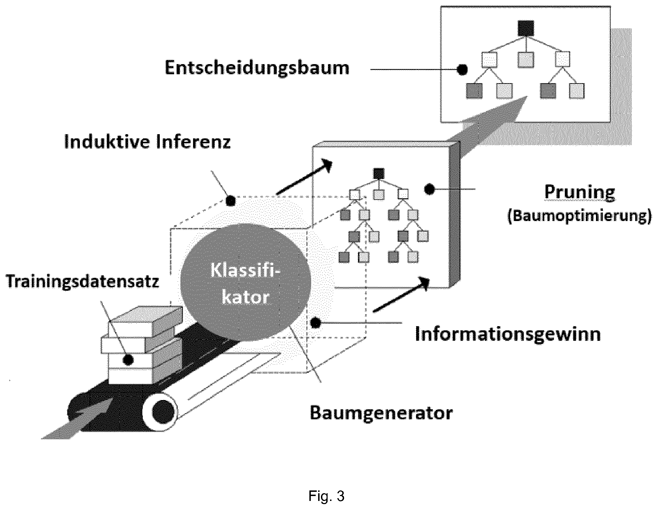

Die Figur 3 zeigt die formalen Komponenten eines Klassifikationsalgorithmus.

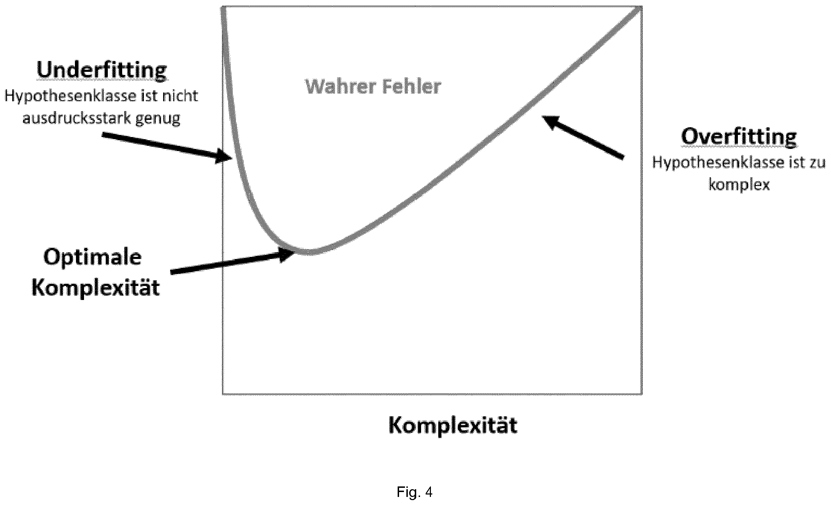

Problem: Der Klassifikator ist im ersten Schritt für die Trainingsdaten optimiert. Er kann auf der Grundgesamtheit der Daten evtl. schlechtere Ergebnisse (Underfitting oder Overfitting: Hypothesenklasse ist zu ausdrucksschwach oder zu komplex) liefern, vergleiche Figur 4.

Ein mögliches Overfitting kann durch **Pruning-oder Boosting-Verfahren** verringert werden, hierbei wählt der Fachmann die Zahl der benötigten Iterationsschritte zur Verbesserung der Gruppenbildung.

Generell teilt man die Menge der vorhandenen Beispiele in zwei Teilmengen (Train-and-Test).

- **Trainingsmenge:** zum Lernen des Klassifikators (Konstruktion des Modells)

- **Testmenge:** zum Bewerten des Klassifikators

Ist dies nicht anwendbar, weil die Menge der Objekte mit bekannter Klassenzugehörigkeit klein ist, so wird anstelle von Train-and-Test die sogenannte m-fache **Cross-Validierung** verwendet.

Als **Gütemaße für Klassifikatoren** nimmt man folgende Kriterien:

- Klassifikationsgenauigkeit

- Kompaktheit des Modells (z.B. Größe eines Entscheidungsbaums)

- Interpretierbarkeit des Modells.

- Effizienz

- Robustheit gegenüber Rauschen und fehlenden Werten

**2.2) Konstruktion von Entscheidungsbäumen**

(Vergleiche auch

- Quinlan, J. Ross (1986): Induction of decision trees. In: Machine Learning 1 (1), S. 81-106.
- Quinlan, J. Ross (1993): C4.5. Programs for machine learning. In: J. Ross Quinlan. San Mateo, Calif.: Morgan Kaufmann (The Morgan Kaufmann series in machine learning).
- Quinlan, J. Ross (1996): Improved use of continuous attributes in C4. 5. Journal of artificial intelligence research 4, S. 77-90.)

**Basis-Algorithmus**

Schleife:

1. Wähle das "Beste" Entscheidungsattribut A für den nächsten Knoten
2. Für jeden Wert von A erzeuge einen neuen Abkömmlingsknoten
3. Ordne die Trainingsdaten den Abkömmlingsknoten zu
4. Wenn die Trainingsdaten fehlerfrei klassifiziert werden, dann STOPPE. Sonst iteriere über Abkömmlingsknoten (→ 1.).

**Bezeichnungen:**

Trainingsdatensatz T,

Anzahl der Trainingsdaten |T|,

Klassen $C_i$: Der Datensatz aller Trainingsdaten in der Klasse $C_i$. $|C_i|$ list die Anzahl der Elemente in Klasse $C_i$. Es gilt: $\Sigma |C_i| = |T|$ (i=1,..k).

Attribut A = {$a_1, a_2, ... , a_m$}. Das Attribut A unterteilt den Datensatz T in m Teilsätze $T_1, T_2, ... ,T_m$. $|T_i|$ ist die Anzahl der Teilmenge $T_i$.

Gegeben Trainingsdatensatz T und Attribute A;

Output: Informationsgewinn(T, A) von Attribute A für den Trainingsdatensatz T

**3) Algorithmus (Berechnung des Informationsgewinns mit Hilfe der Entropie am Beispiel von ID3)**

• Die empirische **Entropie für eine Menge T** von Trainingsobjekten mit den Klassen $C_i$ (i=1,...,k) ist definiert als

$$entropie(T) = \sum_{i=1}^{k} p_i \cdot \log p_i \qquad \text{mit } p_i := |C_i|/|T|$$

• Das Attribut A habe die Partitionierung $T_1, T_2, ..., T_m$ erzeugt. Die empirisch bedingte **Entropie G(T,A) für eine Menge T und ein Attribut A** ist definiert als

$$G(T, A) = \sum_{i=1}^{m} \frac{|T_i|}{|T|} \cdot entropie(T_i)$$

• Der **Informationsgewinn** Gain(T, A) durch das Attribut A in Bezug auf T ist definiert als

$$Gain(T, A) = entropie(T) - G(T, A) = entropie(T) - \sum_{i=1}^{m} \frac{|T_i|}{|T|} \cdot entropie(T_i)$$

**Input:** Trainingsdatensatz T mit den Klassen $C_i$ (i=1, ...,k), Attribute A und Schwellenwert $\varepsilon$ ;

**Output:** Entscheidungsbaum E;

**Algorithmus:**

1. Erstellen eines Knotens K;

2. Falls alle Beispieldaten in T eine identische Klasse $C_j$ haben oder die Anzahl der Daten kleiner als Schwellenwert $\varepsilon$ ist, dann wird der einzige Knoten mit Klasse $C_j$ zum Knoten K als Blatt zurück;

3. Falls A = Ø, dann wird der einzige Knoten mit der häufigsten Klasse in T zum Knoten K als Blatt zugeordnet;

4. Berechnung des Informationsgewinns Gain von A in T und Bestimmung des besten Attributs $A_g$ mit maximalem

Informationsgewinn anhand

$$Gain(T,A) = entropie(T) - G(T,A) = entropie(T) - \sum_{i=1}^{m} \frac{|T_i|}{|T|} \cdot entropie(T_i)$$

5. Bezeichnen den Knoten K mit $A_g$

6. Berechne für alle Attributwerte $A_{gj}$ von $A_g$ den Teildatensatz $T_{gi}$ aller Beispiele aus dem Trainingsdatensatz mit $A_{gj}$

7. Ist $T_{gi} = \emptyset$, dann wird ein Blatt mit der häufigsten Klasse zum Knoten K angefügt andernfalls

8. Rekursion des Zweiges von $A_g$.

**Bemerkung:**

Für eine Zufallsereignis y, welches mit Wahrscheinlichkeit P(y) aufritt, gilt:
**Informationsgehalt:**

$$h(y) \equiv -\log_2(P(y))$$

Die Entropie ist der mittlere Betrag des Informationsgehalts der Zufallsvariable y

$$H[y] \equiv \sum y\, P(y)h(y) = -\sum y\, P(y)\log2(P(y))$$

**Verwendete Klassifikationsalgorithmen**

**ID3**

- Diskrete Attribute, keine fehlenden Attribute

- Information Gain als Qualitätsmaß.

**C4.5/C5.0**

- Erweiterung von ID3.

- Information GainRatio als Qualitätsmaß. Fehlenden Attribute

- Numerische und reellwertige Attribute

- Pruning des Entscheidungsbaumes

Die Information GainRatio ist definiert als

$$GainRatio\,(T,A) := \frac{Gain(T,A)}{SplitInformation(T,A)}$$

$$SplitInformation(T,A) = -\sum_{i=1}^{m} \frac{|T_i|}{|T|} \cdot \log_2\left(\frac{|T_i|}{|T|}\right)$$

**CART (Classification And Regression Trees)**

**Vergleiche auch** Hastie, T., Tibshirani, R., Friedman, J. H. (2001). The elements of statistical learning: Data mining, inference, and prediction. New York: Springer Verlag.

Verfahren analog zu ID3 bzw. C5.0. Das Informationsmaß wird durch den **Gini-Index** definiert. Der Gini Index wird minimiert (anstatt den Gini Gain zu maximieren).

Gini gain

$$Gini(T, A) = \sum_{i=1}^{k} \frac{T_i}{T} \cdot Gini(T_i)$$

$$Gini(T) = 1 - \sum_{i=1}^{k} p_i 2$$

Mit

$P_i$ ist die relative Häufigkeit der Klasse $C_i$ in **T**

***CHAID(Chi*-square*Automatic Interaction* Detectors)**

Vergleiche auch Sonquist, J.A. and Morgan, J.N. (1964): The Detection of Interaction Effects. Survey Research Center, Institute for Social Research, University of Michigan, Ann Arbor.

**CHAID** ist ein weiterer Algorithmus zur Konstruktion von Entscheidungsbäumen. Die Unterschiede zu C5.0 oder CART bestehen darin, dass zur Wahl der Attribute beim CHAID-Algorithmus der Chi-Quadrat-Unabhängigkeitstest verwendet wird und dass der CHAID-Algorithmus das Wachsen des Baumes stoppt, bevor der Baum zu groß geworden ist. Der Baum wird also nicht beliebig wachsen gelassen, um ihn danach mit einer Pruning-Methode wieder zu kürzen.

**4) Radiale Basisfunktionen Netze (RBF-Netze)**

Vergleiche auch Zell, A. : Simulation neuronaler Netze. Oldenbourg 1994

Zur weiteren Validierung der Modelle und zur Berechnung der Wichtigkeit der Biomarker für die verschiedenen Klassifikationsmodelle werden RBF-Netze verwendet. RBF-Netze erstellen Vorhersagemodelle. Sie eignen sich besonders für die Approximation von Funktionen.

Das RBF-Netz besteht aus einer Inputschicht mit n Neuronen, einer Hidden Schicht mit k Neuronen und einer Output-Schicht mit m Neuronen. Ein n-dimenisonales Muster wird hierdurch in einen m-dimensionalen Output-Raum abgebildet.

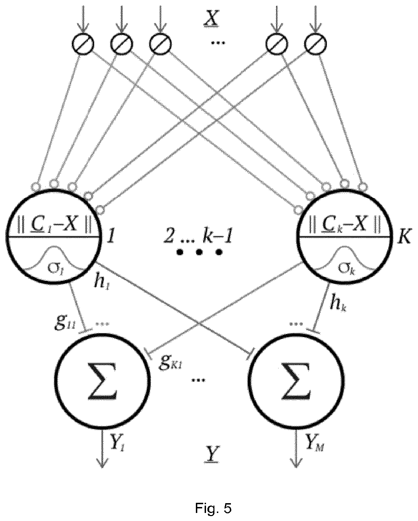

Die Inputschicht ist eine reine Weiterleitung. Jedes Neuron verteilt seinen Wert an alle verdeckten Neuronen. In der Hidden Schicht wird in jedem Neuron der Abstand zwischen der Eingabe und dem Zentrum c mit Hilfe einer euklidischen Norm gebildet. Als Netzeingabe- und Aktivierungsfunktion werden radiale Basisfunktionen verwendet (vergleiche Figur 5).

Die Aktivierungsfunktion jedes versteckten Neurons ist eine sogenannte radiale Funktion, d.h. eine monoton fallende Funktion

$$f : \mathbb{R}_0^+ \to [0, 1] \text{ mit } f(0) = 1 \text{ und } \lim_{x \to \infty} f(x) = 0 \text{m}$$

Die Eingabevektoren werden **normalisiert** (Subtraktion des Mittelwerts und Division durch den Bereich (x-Min)/(Max-Min); Normalisierte Werte liegen im Bereich zwischen 0 und 1). Als Aktivierungsfunktion werden die **Softmax-Funktion** $\sigma$ als normalisierte radiale Basisfunktion verwendet. Softmax $\sigma$ bildet einen k-dimensionalen Vektor z auf einen k-dimensionalen Vektor $\sigma(z)$ ab.

$$\sigma(\mathbf{z})_j = \frac{e^{z_j}}{\sum_{k=1}^{K} e^{z_k}} \text{, j=1, …, k}$$

Die Anzahl der Neuronen in der verdeckten Schicht wird durch das **"Bayesian Information Criterion"**(Vergleiche Schwarz, Gideon E. (1978), "Estimating the dimension of a model", Annals of Statistics, 6 (2): 461-464, MR 468014, doi:10.1214/aos/1176344136) (BIC) bestimmt. Die beste Anzahl an verborgenen Einheiten ist diejenige, die auf der Basis der Trainingsdaten den kleinsten BIC ergibt.

Für die Ausgabeschicht benutzten wir als Aktivierungsfunktion die Identitätsfunktion. Die Ausgabeeinheiten sind also einfach gewichtete Summen der verborgenen Einheiten. Die Ausgabe des Netzwerkes ist daher eine Linearkombination der Radialen Basisfunktionen der Eingaben und der Gewichte.

**Netzleistung**

Die Netzleistung überprüft, wie "gut" das Netz ist. Hierzu werden eine Reihe von Ergebnissen bereitgestellt.

- Modellzusammenfassung.
  Ergebnisse einschließlich Fehler, Relativer Fehler oder Prozentsatz der falschen Vorhersagen und Trainingszeit.

- Klassifikationsergebnisse.
  Für jede kategoriale abhängige Variable wird eine Klassifikationstabelle angegeben.

- ROC-Kurven.
  Die ROC-Kurven (Receiver Operating Characteristic curves) geben die Sensitivität und Spezifität für jeden möglichen Cutpoint der Eingangsvariablen an. Die Area under the Curve AUC ist ein Maß für die Qualität der Klassifikation.

- Kumulative Gewinndiagramme.

**Beispiele**

Die nachfolgenden Beispiele beruhen auf zwei unterschiedlichen Patientenkollektiven. Das Patentenkollektiv 1 wurde als Basis für die nachfolgenden Beispiele 1 und 3 hinzugezogen. Es zeichnet sich durch eine Zahl von **7** Individuen, bei denen bei **4** ein T2D vorlag und das Patentenkollektiv 2, das die Basis für die Beispiele 2, 4 und 5 darstellt, bestand aus **61** Probanden, von denen bei **26** ein T2D vorlag.

Als Kalibrator für die relativen Genexpressionswerte diente eine Probe mit folgenden $\Delta C_T$:

Patientenkollektiv 1 & 2:   *HMGA2:*   6,513
   *ADIPOQ:*   5,484

Die $\Delta C_T$-Werte sind dabei der $C_T$-Wert des Zielgens minus dem $C_T$-Wert der endogenen Kontrolle (housekeeping Gen), wobei der $C_T$-Wert jeweils der Wert ist, bei dem in der Amplifikation erstmalig das Signal für die jeweilige cDNA den Schwellwert überscheitet.

Für die Mittelwerte der relativen Genexpression im Patientenkollektiv 2 ergaben sich folgende Mittelwerte:

*HMGA2:*   1,663117
*ADIPOQ:*   0,95365

**Beispiel 1**

**Nachweis der Expression von *ADIPOQ* und *HMGA2* in Fettgewebspunktaten, gewonnen durch Feinnadel-Aspiration des subkutanen abdominalen Fettgewebes**

**Material und Methoden**

**Feinnadel-Aspiration**

Feinnadel-Aspirate wurden durch Punktion von subkutanem abdominalem hWAT mittels 20 ml Spritze und einer Einmal-Injektions-Kanüle (Durchmesser 0,90 x 40 mm) gewonnen. Nach Desinfektion der Einstichstelle wurde die Kanüle in das Unterhaut-Fettgewebe eingeführt. Mit der Spritze wurde ein Unterdruck erzeugt und die Kanüle im Gewebe fächerförmig vor und zurück bewegt, um so Zellen des Fettgewebes zu aspirieren. Direkt nach der Punktion wurden die Proben in 1 ml QIAzol Lysis Reagenz (QIAGEN, Hilden, Deutschland) aufgenommen und die Kanüle mehrmals mit dem QIAzol gespült. Anschließend wurden die Proben bei -80 °C eingefroren.

**RNA-Isolierung**

Die Isolierung der Gesamt-RNA erfolgte mittels RNeasy Lipid Tissue Mini Kit (QIAGEN, Hilden, Deutschland) in einem QIAcube (QIAGEN, Hilden, Deutschland) nach Herstellerangaben. Die Feinnadel-Aspirate (5mg) in 1 ml QIAzol Lysis Reagenz wurden in einem Tissue Lyser II (QIAGEN, Hilden, Deutschland) homogenisiert und anschließend wurde das Homogenat bei Raumtemperatur für 5 min inkubiert. Es folgte die Zugabe von 200 µl Chloroform, welches mittels kräftigem Schütteln per Hand für 15 sec mit der Probe vermischt wurde. Die Probe wurde erneut für 2 min bei Raumtemperatur inkubiert und mit 12.000 x g zentrifugiert für 15 min bei 4 °C. Anschließend wurde die obere wässrige Phase in ein neues 2 ml Cup transferiert und die Gesamt-RNA wurde über eine Qiagen RNeasy Mini Spin Säule (QIAGEN, Hilden, Deutschland) in einem QIAcube nach Herstellerangaben isoliert.

**cDNA-Synthese**

Für die cDNA-Synthese wurde $\leq 250$ ng RNA mittels 200 U M-MLV Reverse Transkriptase, RNase Out (Thermo Fisher Scientific, Darmstadt, Germany) und 150 ng Random-Primer (Thermo Fisher Scientific, Darmstadt, Germany) nach Herstellerangaben in cDNA umgeschrieben. Die RNA wurde bei 65 °C für 5 min denaturiert und anschließend mindestens 1 min auf Eis gelagert. Nach der Zugabe des Enzyms wurde für das Annealing der Random-Primer an die RNA der Mix für 10 min bei 25 °C inkubiert. Die anschließende Reverse Transkription wurde bei 37 °C für 50 min durchgeführt, gefolgt von einer 15 min Inaktivierung der Reversen Transkriptase bei 70 C.

**Prä-Amplifizierung der cDNA**

5 µl cDNA wurde mittels RealTime ready cDNA Preamp Mastermix (Roche, Mannheim, Germany) unter Verwendung von *HMGA2* und *HPRT* (Hypoxanthin-Phosphoribosyltransferase 1) spezifischen Primern nach Herstellerangaben präamplifiziert. Die genspezifischen Primer binden dabei in geeigneter Weise an die jeweilige cDNA, so dass die erzeugten Amplikons die Bindestellen der Primer der in der quantitativen Real-Time PCR verwendeten genspezifischen Assays enthalten. Die Prä-Amplifikation der cDNA erfolgte nach folgendem Temperaturprofil: 95 °C für 1 min gefolgt von 14 Zyklen bei 95 °C für 15 sec und bei 60 °C für 4 min.

**Quantitative Real-Time PCR** (qRT-PCR)

Die relative Quantifizierung der Genexpression wurde mittels Real-Time PCR auf dem Applied Biosystems 7300 Real-Time PCR System durchgeführt. Kommerziell erhältliche Genexpressions-Assays (Life Technologies, Carlsbad, CA, USA) wurden zur Quantifizierung der mRNA-Level von *HMGA2* (Assay-ID Hs00171569_m1) und *ADIPOQ* (Assay-ID Hs00605917_m1) verwendet. Als endogene Kontrolle wurde, wie von Klemke et al. (Klemke M, Meyer A, Hashemi Nezhad M, Beige G, Bartnitzke S, Bullerdiek J (2010) Loss of let-7 binding sites resulting from truncations of the 3' untranslated region of HMGA2 mRNA in uterine leiomyomas. Cancer Genet Cytogenet 196:119-123) beschrieben, *HPRT* genutzt. Alle gemessenen Proben wurden dreifach bestimmt. Die Quantifizierung der Genexpression wurde in 96-Well Platten mit der zu untersuchenden präamplifizierten cDNA, des jeweiligen genspezifischen Assays und des FastStart Universal Probe Master (Rox) (Roche, Mannheim, Germany) durchgeführt. Das Temperaturprofil der Real-Time PCR folgte den Herstellerangaben: Bei 95 °C erfolgt eine Denaturierung des Templates für 10 min. Anschließend folgte die Amplifikation in 50 Zyklen beginnend mit der Denaturierung für 15 sec bei 95 °C und der Kombination aus Annealing/Elongation für 60 sec bei 60 °C. Die erhaltenen Daten wurden mittels komparativer Delta-Ct-Methode ($\Delta\Delta$CT-Methode) ausgewertet. [(Livak KJ, Schmittgen TD (2001) Analysis of relative gene expression data using real-time quantitative PCR and the 2 -$\Delta\Delta$C(T) Method. Methods 25: 402-408)].

**Ergebnis**

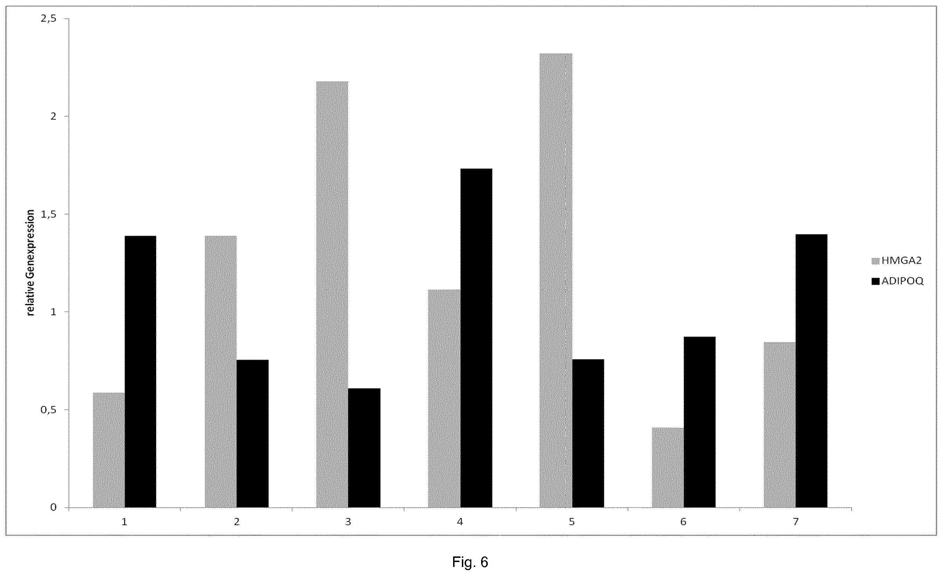

Die Genexpression von *ADIPOQ* und *HMGA2* konnte mittels qRT-PCR in sieben Proben von humanen Patienten gemessen werden (Fig. 6). Die Ergebnisse zeigen eindeutig, dass *ADIPOQ*- und HMGA2-mRNAs zuverlässig aus Feinnadel-Aspiraten mit selbst sehr geringen Mengen an Fettgewebszellen quantifizierbar sind. Zudem reicht die Menge der isolierten RNA aus den Feinnadel-Aspiraten aus, um die Expression von weiteren Genen nach einer Prä-Amplifikation zu bestimmen.

Fig.6 stellt die relative Expression von *HMGA2* und *ADIPOQ* in sieben Proben dar. Sie zeigt, dass eine zuverlässige Quantifizierung der *HMGA2*- und *ADIPOQ*-Expression selbst aus sehr geringen Mengen von RNA (Konzentration ≤ 25 ng/µl), die durch Feinnadel-Aspiration gewonnen wurde, gelingt. Zudem zeigt Fig.6, dass die Expression der Gene zwischen den einzelnen Proben variiert und somit interindividuelle Unterschiede detektierbar sind.

**Beispiel 2**

**Die Expression von *ADIPOQ* in Fettgewebsproben von Typ-2-Diabetikern und Nicht-Diabetikern**

**Material und Methoden**

**Gewebeproben**

Die humanen subkutanen abdominalen Fettgewebe wurden während Operationen entnommen und nach der Operation in flüssigem Stickstoff gelagert. Anschließend wurden die Proben bei -80 °C eingefroren. Für alle verwendeten humanen Fettgewebsproben wurden die Forderungen der Deklaration von Helsinki erfüllt. Eine schriftliche Einverständniserklärung für die Verwendung der Gewebeproben wurde von den Patienten (n = 61) abgegeben.

**RNA-Isolierung**

Die Isolierung der Gesamt-RNA erfolgte mittels RNeasy Lipid Tissue Mini Kit (QIAGEN, Hilden, Deutschland) in einem QIAcube (QIAGEN, Hilden, Deutschland) nach Herstellerangaben. Die Fettgewebsproben (50-100 mg) in 1 ml QIAzol Lysis Reagenz wurden in einem Tissue Lyser II (QIAGEN, Hilden, Deutschland) homogenisiert und anschließend wurde das Homogenat bei Raumtemperatur für 5 min inkubiert. Es folgte die Zugabe von 200 µl Chloroform, welches mittels kräftigem Schütteln per Hand für 15 sec mit der Probe vermischt wurde. Die Probe wurde erneut für 2 min bei Raumtemperatur inkubiert und mit 12.000 x g zentrifugiert für 15 min bei 4 °C. Anschließend wurde die obere wässrige Phase in ein neues 2 ml Cup transferiert und die Gesamt-RNA wurde über eine Qiagen RNeasy Mini Spin Säule (QIAGEN, Hilden, Deutschland) in einem QIAcube nach Herstellerangaben isoliert. Die RNA-Konzentration wurde mittels eines Photometers bestimmt und die Proben wurden anschließend bei -80°C gelagert.

**cDNA-Synthese**

Für die cDNA-Synthese wurde 250 ng RNA mittels 200 U M-MLV Reverse Transkriptase, RNase Out (Thermo Fisher Scientific, Darmstadt, Germany) und 150 ng Random-Primer (Thermo Fisher Scientific, Darmstadt, Germany) nach Herstellerangaben in cDNA umgeschrieben. Die RNA wurde bei 65 °C für 5 min denaturiert und anschließend mindestens 1 min auf Eis gelagert. Nach der Zugabe des Enzyms wurde für das Annealing der Random-Primer an die RNA der Mix für 10 min bei 25 °C inkubiert. Die anschließende Reverse Transkription wurde bei 37 °C für 50 min durchgeführt, gefolgt von einer 15 min Inaktivierung der Reversen Transkriptase bei 70 C.

**Quantitative Real-Time PCR** (qRT-PCR)

Die Durchführung der relativen quantitativen Real-Time PCR erfolgte wie in Beispiel 1 beschrieben.

**Ergebnis**

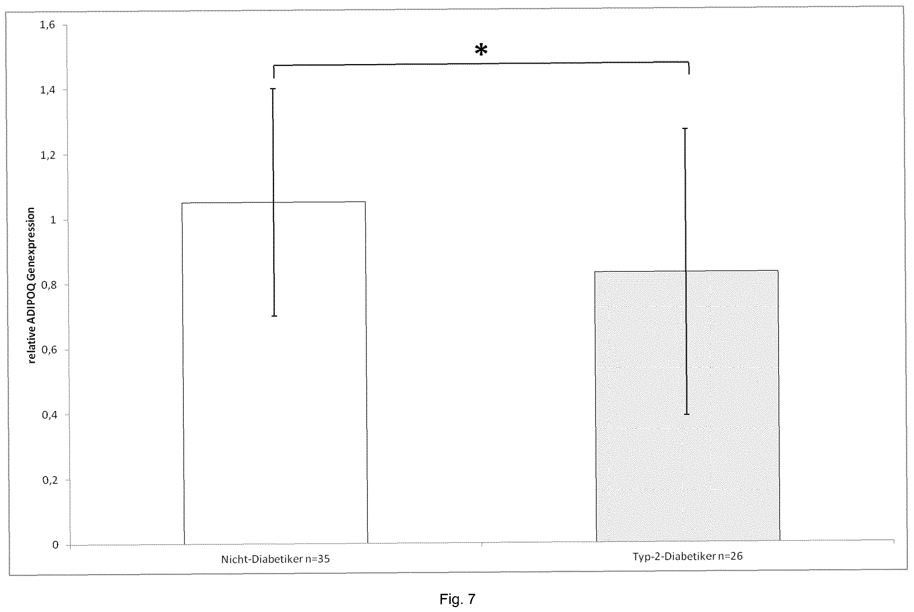

Die in der Literatur beschriebenen Unterschiede des Adiponektinspiegels im Blutplasma von Typ-2-Diabetikern und Nicht-Diabetikern können auch auf Ebene der Genexpression von *ADIPOQ* im Fettgewebe von Typ-2-Diabetikern und Nicht-Diabetikern nachgewiesen werden (Fig. 7). Die Fig. 7 zeigt den signifikanten Unterschied (p < 0,05) der relativen Genexpression von *ADIPOQ* im Fettgewebe von 35 Nicht-Diabetikern und 26 Typ-2-Diabetikern.

**Beispiel 3**

**Unterschiedliche Genexpression von *ADIPOQ* in Fettgewebspunktaten von Typ-2-Diabetikern**

**Material und Methoden**

**Probenvorbereitung**

Die Probengewinnung erfolgte mittels Feinnadel-Aspiration wie in Beispiel 1 beschrieben. Auch die nachfolgenden Schritte der Probenaufbereitung, also RNA-Isolierung, cDNA-Synthese, Prä-Amplifizierung der cDNA und die quantitative Real-Time-PCR erfolgten wie in Beispiel 1 beschrieben.

**Ergebnis**

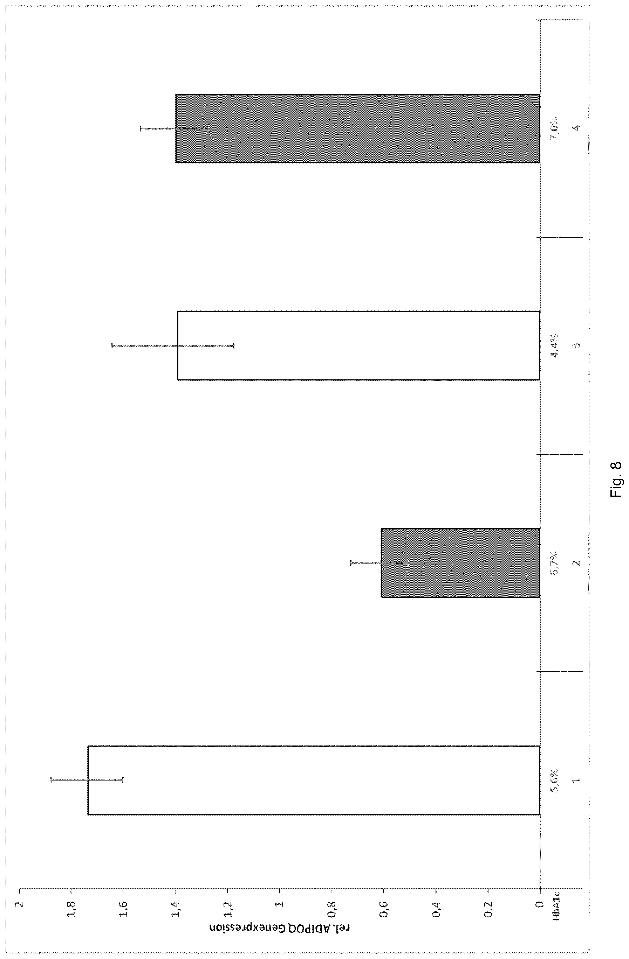

Die Fig. 8 zeigt die Expression von *ADIPOQ* in Fettgewebspunktaten von vier Patienten. Die Patienten 2 und 4 haben einen HbA1c-Wert von 6,7% bzw. 7,0% und sind somit Diabetiker, die Patienten 1 und 3 sind Nicht-Diabetiker (HbA1c-Werte von 5,6% bzw. 4,4%). Die gemessenen ADIPOQ-Genexpressionswerte betragen für den Patienten 1: 1,734; für Patienten 2: 0,609; für Patienten 3: 1,389 und für Patienten 4: 1,397. Die gemessenen Genexpressionen von *ADIPOQ* in Proben von humanen Patienten zeigen (Fig. 8), dass überraschenderweise auch Typ-2-Diabetiker wie Patient 4 eine hohe *ADIPOQ*-Expression aufweisen kann. Dieses Ergebnis ist nach Stand der Literatur nicht zu erwarten gewesen. Die unterschiedliche *ADIPOQ*-Expression bei den Patienten 2 und 4 würde auch eine unterschiedliche Therapieform im Hinblick auf z.B. die Gabe oraler Antidiabetika bedeuten. Das vom ADIPOQ-Gen codierte Protein Adiponektin supprimiert über den Rezeptor AdipoR1 die hepatische Glukoneogenese und stimuliert über den Rezeptor AdipoR2 die Glukoseaufnahme im Skelettmuskel. Daher wären bei hohen *ADIPOQ*-Expressionswerten die Medikamentengabe aus der Gruppe Biguanid bzw. Biguanid-Derivate (z.B. Metformin) nicht empfehlenswert, da diese Wirkstoffgruppe die hepatische Glukoneogenese hemmt und die periphere Insulinwirkung verbessert, somit ähnelt das Wirkprinzip von Biguanid bzw. Biguanid-Derivate dem von Adiponektin. Bei niedrigen *ADIPOQ*-Expressionswerten könnte hingegen die Gabe von Biguanid bzw. Biguanid-Derivate (möglicherweise) empfehlenswert sein, um somit den relativen Mangel an Adiponektin auszugleichen.

**Beispiel 4**

**Die Genexpression von *ADIPOQ* in Fettgewebsproben von normal- und übergewichtigen Individuen**

**Material und Methoden**

**Probenvorbereitung**

Die Fettgewebsproben wurden während Operationen gewonnen wie in Beispiel 2 beschrieben. Auch die nachfolgenden Schritte der Probenaufbereitung, also RNA-Isolierung, cDNA-Synthese und die quantitative Real-Time-PCR erfolgten wie in Beispiel 2 beschrieben.

**Ergebnis**

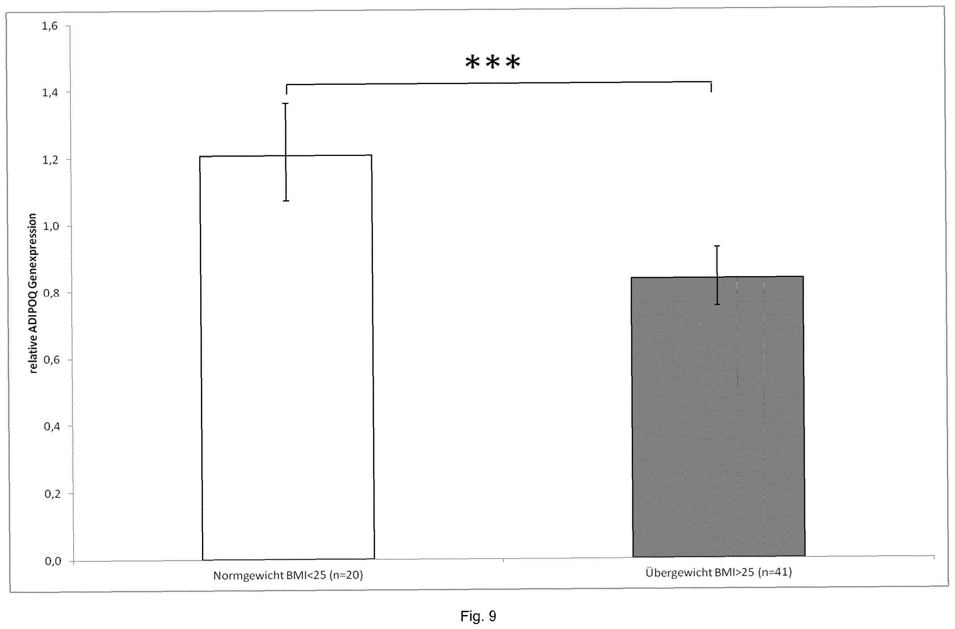

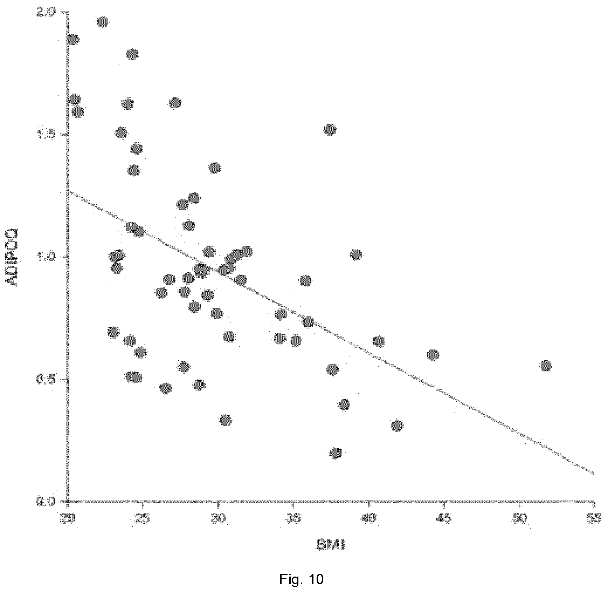

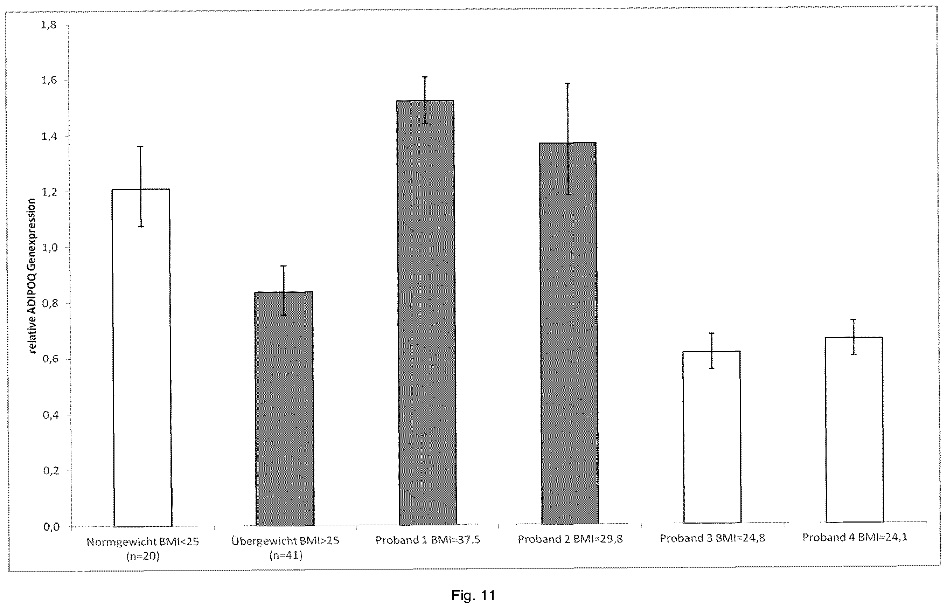

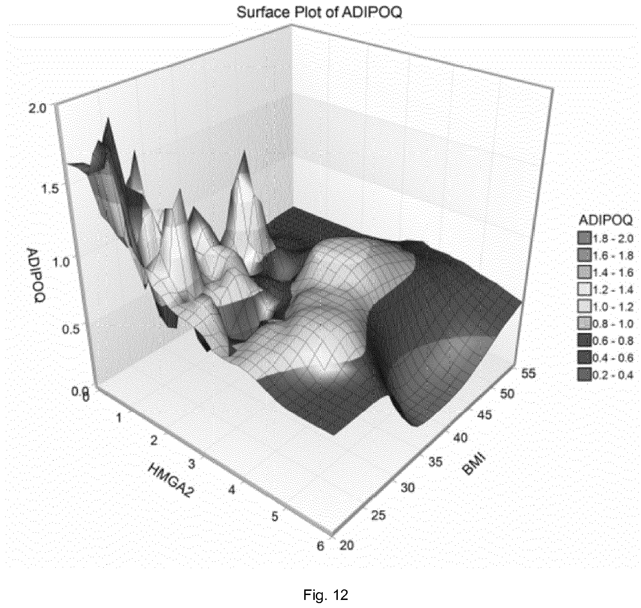

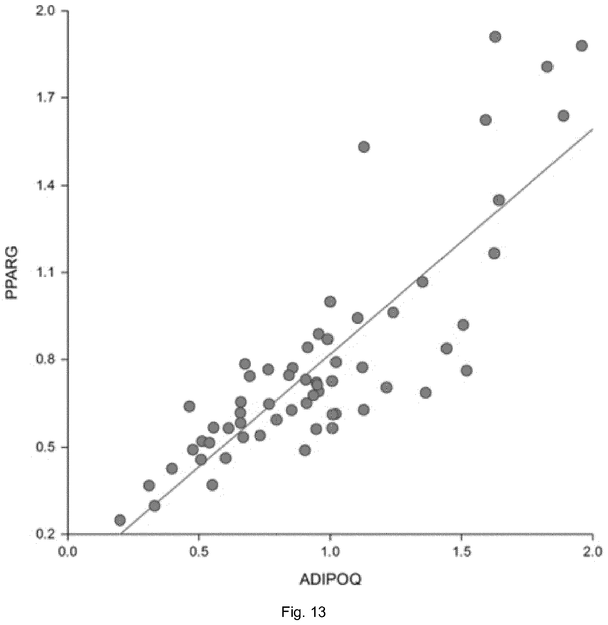

Die Fig. 9 zeigt die Expression von *ADIPOQ* in Fettgewebsproben von 20 Patienten mit Normalgewicht (BMI < 25) und 41 Patienten mit Übergewicht (BMI $\geq$ 25). Die Genexpression von *ADIPOQ* in der Gruppe der Normalgewichtigen betrug 1,207 und war höchst signifikant höher ($p < 0,001$) als die *ADIPOQ*-Expression in der Gruppe der Übergewichtigen mit 0,834. Die Analyse der Korrelation von *ADIPOQ*-Expression und BMI an 61 Patienten zeigte einen mittelstarken (-0,507) und höchst signifikanten ($p < 0,001$) inversen Zusammenhang (Fig. 10). Dieses Ergebnis stimmt grundsätzlich mit dem in der Literatur beschriebenen inversen Zusammenhang zwischen Adiponektinspiegel im Blutplasma und dem Körpergewicht überein. Vergleicht man aber die Genexpression von *ADIPOQ* von Individuen mit Übergewicht oder Adipositas bzw. Normalgewichtige mit der *ADIPOQ*-Expression des Gesamtpatientenkollektivs (s. Fig. 11), so zeigt sich überraschenderweise, dass es Ausnahmen zwischen dem inversen Zusammenhang zwischen Adiponektinspiegel im Blutplasma und dem Körpergewicht besteht. Die Fig. 6 zeigt, dass der männliche Proband 1 mit einem BMI von 37,5 und die weibliche Probandin 2 (BMI = 29,8) eine *ADIPOQ*-Expression von 1,519 bzw. 1,364 aufweisen, die eher der *ADIPOQ*-Expression der Gruppe der Normalgewichtigen mit 1,207 entspricht. Zudem zeigen die normalgewichtigen männlichen Probanden 3 (BMI=24,8) und 4 (BMI=24,1) eine *ADIPOQ*-Expression von 0,612 bzw. 0,659 welche im Bereich der *ADIPOQ*-Expression der Übergewichtigen mit 0,834 liegt. Diese Daten legen nahe, dass die inverse Korrelation von *ADIPOQ*-Expression und BMI noch mit einem weiteren Faktor korrespondiert. Die Patentschrift DE10 2015 208 083 B3 offenbart einen Zusammenhang zwischen *HMGA2*-Expressionslevel und dem BMI. Eine Analyse der *ADIPOQ*- und *HMGA2*-Expressionslevel und dem BMI mittels Surface-Plot, wie in Fig. 12 dargestellt, zeigt, dass die Korrelation von *ADIPOQ*-Expressionslevel und BMI anhängig vom *HMGA2*-Expressionslevel ist. Dieser Zusammenhang war aufgrund der wissenschaftlichen Datenlage und nach dem Stand der Technik nicht zu erwarten. Die Patentschrift DE10 2015 208 083 B3 offenbart zudem eine Korrelation zwischen hohen *HMGA2*-Expressionslevel, niedrigen *PPAR-gamma*-Expressionslevel und einem hohen BMI. Da der Peroxisom-Proliferator-aktivierte Rezeptor gamma (PPAR-gamma) Adipokine, wie u.a. Adiponektin, reguliert, wurde die Genexpression von *PPAR-gamma* und *ADIPOQ* untersucht. Es zeigte sich, wie in Fig. 13 dargestellt, eine starke (0,838) und höchst signifikante ($p < 0,001$) Korrelation zwischen der Genexpression von *PPAR-gamma* und *ADIPOQ*.

**Beispiel 5**

**Datenanalyse mittels Selbstorganisierender Karten zeigt fünf unterschiedliche Cluster innerhalb des Patientenkollektivs**

**Material und Methoden**

**Probenvorbereitung**

Die Fettgewebsproben wurden während Operationen gewonnen wie in Beispiel 2 beschrieben. Auch die nachfolgenden Schritte der Probenaufbereitung, also RNA-Isolierung, cDNA-Synthese und die quantitative Real-Time-PCR erfolgten wie in Beispiel 2 beschrieben.

**Statistische Analyse**

Siehe oben, Methodik

**Ergebnis**

Diverse Studien haben in der Vergangenheit gezeigt, dass niedrige Adiponektin-Spiegel im Blut mit einer erhöhten Inzidenz für Typ-2-Diabetes (T2D) einhergehen. Adiponektin gilt als "Schutzfaktor" vor T2D und koronarer Herzkrankheit, hohe Spiegel des Hormons werden als Indikator für ein geringeres Krankheitsrisiko gesehen. Wie die Beispiele 3 und 4 jedoch zeigen, sind hohe *ADIPOQ*-Expressionslevel auch bei Patienten mit T2D und Übergewicht zu finden. Diese Erkenntnisse waren aufgrund der wissenschaftlichen Datenlage nicht zu erwarten und haben somit potentielle Auswirkungen auf eine Behandlung dieser Patienten. Um eine personalisierte Behandlung von z.B. Patienten mit T2D zu ermöglichen, ist es sinnvoll den speziellen Subtyp des T2D, an dem der Patient leidet, zu identifizieren. Ein spezieller T2D-Subtyp könnte z.B. mit einer Insulinresistenz verbunden sein, ein anderer Subtyp könnte mit Problemen in der Insulinsekretion assoziiert sein. Um diese T2D-Subtypen zu identifizieren kann erfindungsgemäß die Analyse der Beziehung der Biomarker *ADIPOQ, HMGA2* und dem Alter mittels Kohonens Selbstorganisierenden Karten (SOM) helfen.

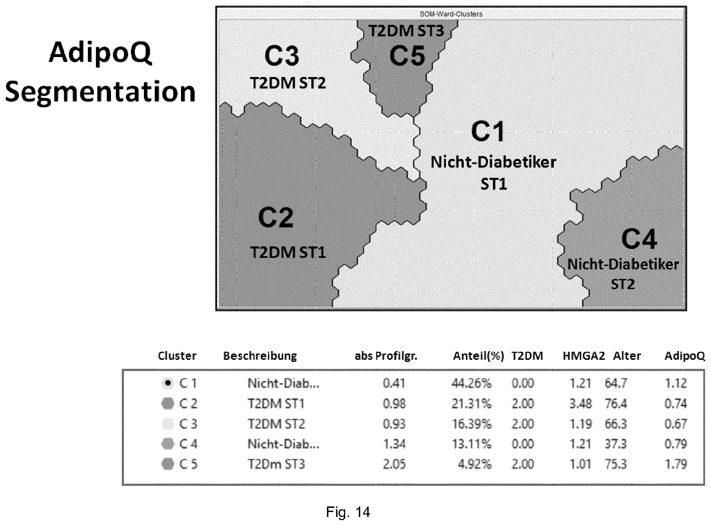

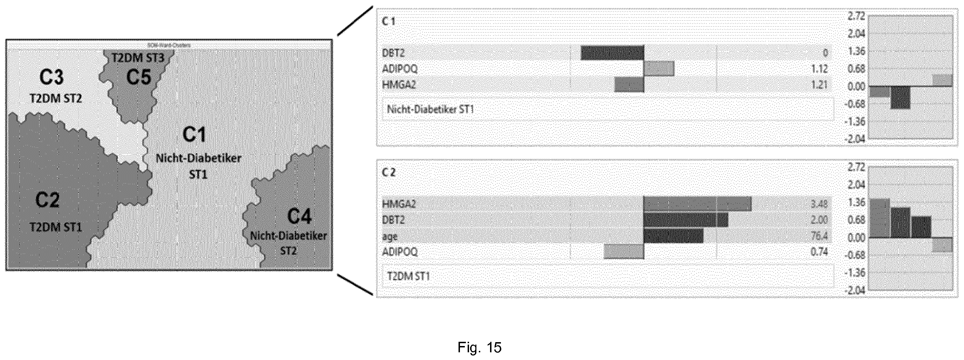

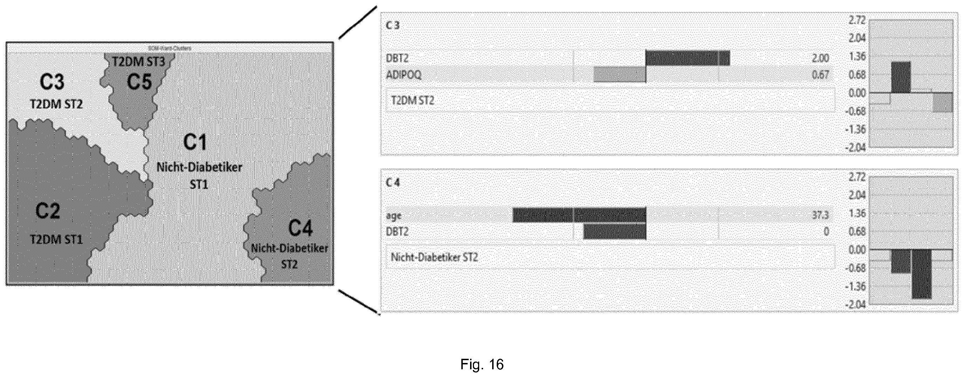

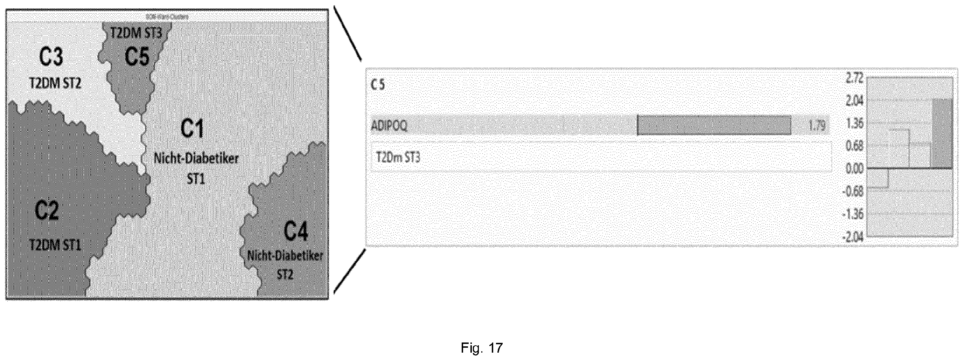

Die Fig. 14 zeigt nach Analyse der Biomarker *ADIPOQ, HMGA2* und dem Alter (als weiteren Marker) mittels SOM überraschenderweise eine Einteilung des Patientenkollektivs in fünf Gruppen und nicht wie man z.B. nach einem HbA1c-Test erwarten würde in zwei Gruppen, nämlich Typ-2-Diabetiker und Nicht-Diabetiker. Die zugehörigen Daten finden sich in der Tabelle 2 und in den Figuren 15-17. Die Einteilung des Patientenkollektivs in fünf Gruppen überrascht auch insofern, da die Patentschrift DE10 2015 208 083 B3 eine Einteilung der Patienten in vier Gruppen offenbarte, nämlich zwei Übergewichtige- und zwei Normalgewichtige-Gruppen.

Die Datenanalyse mittels Selbstorganisierender Karten unterteilt das Probandenkollektiv unter Berücksichtigung der Parameter *HMGA2*- und *ADIPOQ*-Expression in fünf Gruppen. Dabei verteilen sich die Nicht-Diabetiker in zwei Cluster (C1 und C4). Die mittlere *HMGA2*-Expression in beiden Clustern ist gegenüber der mittleren *HMGA2*-Expression des Gesamtkollektivs erniedrigt. Die mittlere *ADIPOQ*-Expression in Cluster C1 ist gegenüber dem Gesamtkollektiv erhöht, in Cluster C4 dagegen erniedrigt. Die Diabetiker untergliedern sich in die Cluster C2, C3 und C5. Die mittlere *HMGA2*-Expression ist in Cluster C2 stark erhöht, die *ADIPOQ*-Expression aber verringert. In Cluster C3 sind die Expressionen beider Gene erniedrigt. Das dritte Diabetiker-Cluster C5 weist eine erniedrigte *HMGA2*-Expression auf, die *ADIPOQ*-Expression ist in diesem Cluster stark erhöht.

***ADIPOQ* Segmentation**

**Tabelle 2**

| Cluster | Bezeichnung | Mittelwert *HMGA2* | Abw. [%] | *HMGA2* | Mittelwert *ADIPOQ* | Abw. [%] | *ADIPOQ* | Mittelwert Alter |
|---------|-------------|--------------------|----------|---------|---------------------|----------|----------|------------------|
| C1 | Nicht-Diabetiker ST1 | 1.21 | -27.2 | erniedrigt | 1.12 | 17.4 | erhöht | 64,7 |

(fortgesetzt)

| Cluster | Bezeichnung | Mittelwert *HMGA2* | Abw. [%] | *HMGA2* | Mittelwert *ADIPOQ* | Abw. [%] | *ADIPOQ* | Mittelwert Alter |
|---|---|---|---|---|---|---|---|---|
| C2 | T2D ST1 | 3.48 | 109.2 | stark erhöht | 0.74 | -22.4 | erniedrigt | 76,4 |
| C3 | T2D ST2 | 1.19 | -28.4 | erniedrigt | 0.67 | -29.7 | erniedrigt | 66,3 |
| C4 | Nicht-Diabetiker ST2 | 1.21 | -27.2 | erniedrigt | 0.79 | -17.2 | erniedrigt | 37,3 |
| C5 | T2D ST3 | 1.01 | -39.3 | erniedrigt | 1.79 | 87.7 | stark erhöht | 75,3 |

Ohne an eine Theorie gebunden zu sein, gibt es folgende biologische Erklärungen für die Cluster:

C2 (T2D Subtyp 1): dysfunktionales Fettgewebe aufgrund vieler Präadipozyten (hoher *HMGA2*-Wert u. verhältnismäßig niedriger *ADIPOQ*-Wert), eher "Insulin-resistenter" T2D-Subtyp. Eine Differenzierung der Präadipozyten hin zu reifen Insulin-sensitiven Adipozyten wäre hier wünschenswert, hier könnten z.B. Medikamente der Gruppe der Glitazone & Metformin eingesetzt werden. Es gibt jedoch Hinweise, dass Insulin und Insulinähnliche Wachstumsfaktoren die Differenzierung von Präadipozyten in Richtung reife Insulin-sensitive Adipozyten fördern (Ayoubi et al., 1999; Klemm et al., 2001). Daher wäre es auch denkbar Insulin und Insulinproduktion-fördernde Medikamente bei hohen *HMGA2*-Werten zu verordnen.

C5 (T2D Subtyp 3): funktionales Fettgewebe aufgrund vieler reifer Insulin-sensitiver Adipozyten (hohe *ADIPOQ*-Werte), erwartbar ein generell "Insulin-sensitiver T2D-Subtyp". Dieser T2D-Subtyp hat vermutlich ein Problem in der Insulin-Produktion/Sekretion in den β-Zellen des Pankreas, daher könnte eine Behandlung hier auf die Verbesserung/Erhöhung der Insulin-Produktion abzielen (z.B. Sulfonylharnstoff und Glinide).

C3 (T2D Subtyp 2): dysfunktionales Fettgewebe vermutlich aufgrund gestörter (zumindest aber zu niedriger) Adipokine-Produktion (mittlerer *HMGA2*-Wert (eher unauffällig) u. sehr niedriger *ADIPOQ*-Wert), vermutlich eher "Insulin-resistenter" T2D-Subtyp. Da hier vermutlich nicht zu viele Präadipozyten vorliegen, könnte die Gabe von Metformin helfen die *ADIPOQ*-Werte zu erhöhen (s. Kubota et al. 2006 und Adamia et al. 2007).

C1 (Nicht-Diabetiker Subtyp 1): spiegelt funktionales Fettgewebe im Hinblick auf Fettzell-Zusammensetzung (Verhältnis Präadipozyten zu Adipozyten vermutlich im "normalen/gesunden" Bereich) als auch Funktion bzw. Adipokin-Produktion ("mittelhohe" *ADIPOQ*-Werte) wider.

C4 (Nicht-Diabetiker Subtyp 2): spiegelt eher ein funktionales Fettgewebe im Hinblick auf die Fettzell-Zusammensetzung (Verhältnis Präadipozyten zu Adipozyten vermutlich im "normalen/gesunden" Bereich) wider. Die im Vergleich zum Cluster C1 niedrigeren *ADIPOQ*-Werte könnten evtl. im Zusammenhang mit dem Alter stehen (der Grund könnte aber auch die geringere Anzahl der Probanden im C4-Cluster sein).

**Patentansprüche**

1. Verfahren zur Prognose und/oder Diagnose einer Diabetes mellitus Typ II-Erkrankung eines Probanden, umfassend die Schritte:

   a) Bestimmen des Genexpressionslevels des Genes *ADIPOQ* in einer Probe des Probanden und
   b) Einordnen des Probanden unter Berücksichtigung des Genexpressionslevels des Genes *ADIPOQ* in eine von wenigstens drei Risikogruppen,
   wobei zum Einordnen des Probanden in Schritt b) ferner das Genexpressionslevel des Genes *HMGA2* berücksichtigt wird,
   und wobei als mögliche Gruppen für eine Einordnung in Schritt b) die Gruppen
   III) stark erhöhtes relatives Genexpressionslevel für *ADIPOQ,* erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq 45$,
   IV) erhöhtes relatives Genexpressionslevel für *ADIPOQ,* erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\geq$ 45,
und

V) erniedrigtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter $\leq$ 45, zur Verfügung stehen.

2.  Verfahren nach Anspruch 1 wobei bei der Eingruppierung in Schritt b) ferner eines oder mehrere Merkmale des Probanden ausgewählt aus der Gruppe bestehend aus BMI, Größe, Gewicht, Geschlecht, Bauch- & Hüftumfang, Körperfettanteil, Muskelmasse, Total Body Water (TBW), Blutdruck, Raucherstatus, Bluthochdruck, Einnahme blutdrucksenkender Medikamente berücksichtigt wird.

3.  Verfahren nach einem der Ansprüche 1 bis 2, wobei die Probe aus Fettgewebe gewonnen wurde.

4.  Verfahren nach Anspruch 3, wobei die Probe durch Punktion des subkutanen abdominalen Fettgewebes gewonnen wurde.

5.  Verfahren nach einem der Ansprüche 1 bis 4, wobei der Proband ein Mensch ist.

6.  Verfahren nach einem der Ansprüche 1 bis 5, wobei die Bestimmung des Genexpressionslevels auf mRNA-Ebene erfolgt.

7.  Verfahren nach einem der Ansprüche 1 bis 6, wobei die Eingruppierung in Schritt b) unter Verwendung des multivariaten Modells der selbstorganisierenden Karten nach Kohonen erfolgt.

8.  Verfahren nach einem der Ansprüche 1 bis 7, wobei die Einordnung in Schritt b) in eine von wenigstens 5 Gruppen erfolgt, wobei wenigstens zwei der Gruppen aus Individuen bestehen, die eine erhöhte Wahrscheinlichkeit der Ausbildung eines Diabetes mellitus Typ II besitzen.

9.  Verfahren nach einem der Ansprüche 1 bis 8, wobei der Genexpressionslevel auf mRNA-Ebene relativ zu dem Genexpressionslevel eines Housekeeping Gens gemessen wird.

10. Verfahren nach einem der Ansprüche 1 bis 9, wobei als mögliche Gruppen für eine Einordnung in Schritt b) die Gruppen

I) erniedrigtes relatives Genexpressionslevel für *ADIPOQ*, stark erhöhtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 45,
II) erniedrigtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 45,
III) stark erhöhtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus erhöhte HbA1c-Blutwerte, erhöhte Cholesterinwerte, erhöhte Triglyceride, erniedrigtes HDL-Cholesterin, erhöhtes non-HDL-Cholesterin, erhöhtes LDL-Cholesterin, erhöhtes CRP, erhöhter Blutzucker, erhöhter Nüchternblutzucker, erhöhter präprandialer Blutzucker, erhöhter postprandialer Blutzucker und Alter $\geq$ 45,
IV) erhöhtes relatives Genexpressionslevel für *ADIPOQ*, erniedrigtes relatives Genexpressionslevel für *HMGA2* und sowie wenigstens eine der Markersituationen ausgewählt aus der Gruppe bestehend aus nicht erhöhte HbA1c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter ≥ 45,
und
V) erniedrigtes relatives Genexpressionslevel für *ADIPOQ,* erniedrigtes relatives Genexpressionslevel für *HMGA2* und sowie wenigstens eine der Markersituationen ausgewählt aus der Gruppe bestehend aus nicht erhöhte HbA1 c-Blutwerte, nicht erhöhte Cholesterinwerte, nicht erhöhte Triglyceride, erhöhtes HDL-Cholesterin, nicht erhöhtes non-HDL-Cholesterin, nicht erhöhtes LDL-Cholesterin, nicht erhöhtes CRP, nicht erhöhter Blutzucker, nicht erhöhter Nüchternblutzucker, nicht erhöhter präprandialer Blutzucker, nicht erhöhter postprandialer Blutzucker und Alter ≤ 45, zur Verfügung stehen.

## Claims

1. Method for prognosing and/or diagnosing a type II diabetes mellitus disease of a subject, comprising the steps of:

   a) determining the gene expression level of the *ADIPOQ* gene in a sample of the subject and
   b) classifying the subject into one of at least three risk groups, taking the gene expression level of the *ADIPOQ* gene into account,
   wherein the gene expression level of the *HMGA2* gene is further taken into account for the classification of the subject in step b),
   and wherein the groups
   III) greatly increased relative gene expression level for *ADIPOQ,* lowered relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased preprandial blood sugar, increased postprandial blood sugar and age ≥ 45,
   IV) increased relative gene expression level for *ADIPOQ,* lowered relative gene expression level for *HMGA2* and also at least one of the marker situations selected from the group consisting of nonincreased HbAlc blood values, nonincreased cholesterol values, nonincreased triglycerides, increased HDL cholesterol, nonincreased non-HDL cholesterol, nonincreased LDL cholesterol, nonincreased CRP, nonincreased blood sugar, nonincreased fasting blood sugar, nonincreased preprandial blood sugar, nonincreased postprandial blood sugar and age ≥ 45,
   and
   V) lowered relative gene expression level for *ADIPOQ,* lowered relative gene expression level for *HMGA2* and also at least one of the marker situations selected from the group consisting of nonincreased HbAlc blood values, nonincreased cholesterol values, nonincreased triglycerides, increased HDL cholesterol, nonincreased non-HDL cholesterol, nonincreased LDL cholesterol, nonincreased CRP, nonincreased blood sugar, nonincreased fasting blood sugar, nonincreased preprandial blood sugar, nonincreased postprandial blood sugar and age ≤ 45 are available as possible groups for a classification in step b).

2. Method according to Claim 1, wherein one or more features of the subject selected from the group consisting of BMI, height, weight, sex, abdominal and hip circumference, body fat percentage, muscle mass, total body water (TBW), blood pressure, smoker status, high blood pressure and ingestion of antihypertensive drugs are further taken into account in the classification in step b).

3. Method according to any of Claims 1 to 2, wherein the sample was obtained from adipose tissue.

4. Method according to Claim 3, wherein the sample was obtained by puncture of subcutaneous abdominal adipose tissue.

5. Method according to any of Claims 1 to 4, wherein the subject is a person.

6. Method according to any of Claims 1 to 5, wherein the determination of the gene expression level is done at the mRNA level.

7. Method according to any of Claims 1 to 6, wherein the classification in step b) is done using the multivariate model of self-organizing maps by Kohonen.

8. Method according to any of Claims 1 to 7, wherein the classification in step b) is done into one of at least 5 groups, at least two of the groups consisting of individuals who have an increased probability of forming type II diabetes mellitus.

9. Method according to any of Claims 1 to 8, wherein the gene expression level is measured at the mRNA level relative to the gene expression level of a housekeeping gene.

10. Method according to any of Claims 1 to 9, wherein the groups

I) lowered relative gene expression level for *ADIPOQ,* greatly increased relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased preprandial blood sugar, increased postprandial blood sugar and age $\geq$ 45,
II) lowered relative gene expression level for *ADIPOQ,* lowered relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased preprandial blood sugar, increased postprandial blood sugar and age $\geq$ 45,
III) greatly increased relative gene expression level for *ADIPOQ,* lowered relative gene expression level for *HMGA2* and also at least one of the marker situations selected from the group consisting of increased HbAlc blood values, increased cholesterol values, increased triglycerides, lowered HDL cholesterol, increased non-HDL cholesterol, increased LDL cholesterol, increased CRP, increased blood sugar, increased fasting blood sugar, increased preprandial blood sugar, increased postprandial blood sugar and age $\geq$ 45,
IV) increased relative gene expression level for *ADIPOQ,* lowered relative gene expression level for *HMGA2* and also at least one of the marker situations selected from the group consisting of nonincreased HbAlc blood values, nonincreased cholesterol values, nonincreased triglycerides, increased HDL cholesterol, nonincreased non-HDL cholesterol, nonincreased LDL cholesterol, nonincreased CRP, nonincreased blood sugar, nonincreased fasting blood sugar, nonincreased preprandial blood sugar, nonincreased postprandial blood sugar and age $\geq$ 45,
and
V) lowered relative gene expression level for *ADIPOQ,* lowered relative gene expression level for *HMGA2* and also at least one of the marker situations selected from the group consisting of nonincreased HbAlc blood values, nonincreased cholesterol values, nonincreased triglycerides, increased HDL cholesterol, nonincreased non-HDL cholesterol, nonincreased LDL cholesterol, nonincreased CRP, nonincreased blood sugar, nonincreased fasting blood sugar, nonincreased preprandial blood sugar, nonincreased postprandial blood sugar and age $\leq$ 45 are available as possible groups for a classification in step b).

**Revendications**

1. Procédé de pronostic et/ou de diagnostic d'un diabète sucré de type II chez un sujet, comprenant les étapes :

a) de détermination du niveau d'expression du gène ADIPOQ dans un échantillon du sujet, et
b) de classification du sujet en tenant compte du niveau d'expression du gène ADIPOQ dans un d'au moins trois groupes de risque,
dans lequel en outre le niveau d'expression du gène HMGA2 est pris en compte pour la classification du sujet dans l'étape b),
et dans lequel sont disponibles, en tant que groupes possibles pour une classification dans l'étape b), les groupes
III) niveau d'expression de gène relatif très élevé pour ADIPOQ, niveau d'expression de gène relatif faible pour HMGA2 ainsi qu'au moins une des situations de marqueur choisie parmi le groupe constitué de taux sanguins de HbAlc élevés, de taux élevés de cholestérol, de triglycérides élevés, d'un taux de cholestérol HDL faible, d'un taux de cholestérol non HDL élevé, d'un taux de cholestérol LDL élevé, d'une CRP élevée, de glycémie élevée, de glycémie à jeun élevée, de glycémie préprandiale élevée, de glycémie postprandiale élevée et d'un âge $\geq$ 45 ans,

IV) niveau d'expression de gène relatif élevé pour ADIPOQ, niveau d'expression de gène relatif faible pour HMGA2 ainsi qu'au moins une des situations de marqueur choisie parmi le groupe constitué de taux sanguins de HbAlc non élevés, de taux non élevés de cholestérol, de triglycérides non élevés, d'un taux de cholestérol HDL élevé, d'un taux de cholestérol non HDL non élevé, d'un taux de cholestérol LDL non élevé, d'une CRP non élevée, de glycémie non élevée, de glycémie à jeun non élevée, de glycémie préprandiale non élevée, de glycémie postprandiale non élevée et d'un âge $\geq$ 45 ans,
et

V) niveau d'expression de gène relatif faible pour ADIPOQ, niveau d'expression de gène relatif faible pour HMGA2 ainsi qu'au moins une des situations de marqueur choisie parmi le groupe constitué de taux sanguins de HbAlc non élevés, de taux non élevés de cholestérol, de triglycérides non élevés, d'un taux de cholestérol HDL élevé, d'un taux de cholestérol non HDL non élevé, d'un taux de cholestérol LDL non élevé, d'une CRP non élevée, de glycémie non élevée, de glycémie à jeun non élevée, de glycémie préprandiale non élevée, de glycémie postprandiale non élevée et d'un âge $\leq$ 45 ans.

2. Procédé selon la revendication 1, dans lequel sont pris en compte, lors du classement dans l'étape b) en outre un ou plusieurs paramètres du sujet choisis parmi le groupe constitué de l'IMC, de la taille, du poids, du sexe, de la circonférence abdominale et du tour de hanches, de la masse de graisse corporelle, de la masse musculaire, du taux de masse hydrique, de la pression artérielle, du statut de fumeur, de l'hypertension, de la prise de médicaments antihypertenseurs.

3. Procédé selon l'une quelconque des revendications 1 à 2, dans lequel l'échantillon a été obtenu à partir de tissu adipeux.

4. Procédé selon la revendication 3, dans lequel l'échantillon a été obtenu par ponction de tissu adipeux abdominal sous-cutané.

5. Procédé selon l'une quelconque des revendications 1 à 4, dans lequel le sujet est un homme.

6. Procédé selon l'une quelconque des revendications 1 à 5, dans lequel la détermination du niveau d'expression de gène est effectuée sur le plan de l'ARNm.

7. Procédé selon l'une quelconque des revendications 1 à 6, dans lequel le classement dans l'étape b) est effectué en utilisant le modèle multivarié des cartes auto-organisatrices de Kohonen.

8. Procédé selon l'une quelconque des revendications 1 à 7, dans lequel la classification dans l'étape b) est effectuée dans un d'au moins 5 groupes, dans lequel au moins deux des groupes sont constitués d'individus, qui possèdent une probabilité élevée de développer un diabète sucré de type II.

9. Procédé selon l'une quelconque des revendications 1 à 8, dans lequel le niveau d'expression de gène est mesuré sur le plan de l'ARNm par rapport au niveau d'expression de gène d'un gène domestique.

10. Procédé selon l'une quelconque des revendications 1 à 9, dans lequel sont disponibles en tant que groupes possibles pour un classement dans l'étape b) les groupes

I) niveau d'expression de gène relatif faible pour ADIPOQ, niveau d'expression de gène relatif très élevé pour HMGA2 ainsi qu'au moins une des situations de marqueur choisie parmi le groupe constitué de taux sanguins de HbAlc élevés, de taux élevés de cholestérol, de triglycérides élevés, d'un taux de cholestérol HDL faible, d'un taux de cholestérol non HDL élevé, d'un taux de cholestérol LDL élevé, d'une CRP élevée, de glycémie élevée, de glycémie à jeun élevée, de glycémie préprandiale élevée, de glycémie postprandiale élevée et d'un âge $\geq$ 45 ans,
II) niveau d'expression de gène relatif faible pour ADIPOQ, niveau d'expression de gène relatif faible pour HMGA2 ainsi qu'au moins une des situations de marqueur choisie parmi le groupe constitué de taux sanguins de HbAlc élevés, de taux élevés de cholestérol, de triglycérides élevés, d'un taux de cholestérol HDL faible, d'un taux de cholestérol non HDL élevé, d'un taux de cholestérol LDL élevé, d'une CRP élevée, de glycémie élevée, de glycémie à jeun élevée, de glycémie préprandiale élevée, de glycémie postprandiale élevée et d'un âge $\geq$ 45 ans,
III) niveau d'expression de gène très élevé pour ADIPOQ, niveau d'expression de gène relatif faible pour HMGA2 ainsi qu'au moins une des situations de marqueur choisie parmi le groupe constitué de taux sanguins de HbAlc élevés, de taux élevés de cholestérol, de triglycérides élevés, d'un taux de cholestérol HDL faible, d'un taux de cholestérol non HDL élevé, d'un taux de cholestérol LDL élevé, d'une CRP élevée, de glycémie élevée, de glycémie à jeun élevée, de glycémie préprandiale élevée, de glycémie postprandiale élevée et d'un âge $\geq$ 45 ans,

IV) niveau d'expression de gène relatif élevé pour ADIPOQ, niveau d'expression de gène relatif faible pour HMGA2 ainsi qu'au moins une des situations de marqueur choisie parmi le groupe constitué de taux sanguins de HbAlc non élevés, de taux non élevés de cholestérol, de triglycérides non élevés, d'un taux de cholestérol HDL élevé, d'un taux de cholestérol non HDL non élevé, d'un taux de cholestérol LDL non élevé, d'une CRP non élevée, de glycémie non élevée, de glycémie à jeun non élevée, de glycémie préprandiale non élevée, de glycémie postprandiale non élevée et d'un âge $\geq$ 45 ans,

et

V) niveau d'expression de gène relatif faible pour ADIPOQ, niveau d'expression de gène relatif faible pour HMGA2 ainsi qu'au moins une des situations de marqueur choisie parmi le groupe constitué de taux sanguins de HbAlc non élevés, de taux non élevés de cholestérol, de triglycérides non élevés, d'un taux de cholestérol HDL élevé, d'un taux de cholestérol non HDL non élevé, d'un taux de cholestérol LDL non élevé, d'une CRP non élevée, de glycémie non élevée, de glycémie à jeun non élevée, de glycémie préprandiale non élevée, de glycémie postprandiale non élevée et d'un âge $\leq$ 45 ans.

| T2D | HMGA2 | PPARG | ADIPOQ | IL6 |
|-----|-------|-------|--------|------|
| 2 | 1,17 | 0,43 | 0,40 | 7,63 |
| 0 | 2,16 | 0,77 | 0,86 | 0,93 |
| 0 | 1,23 | 0,61 | 1,02 | 1,49 |
| 2 | 3,30 | 0,59 | 0,80 | 2,48 |
| 2 | 3,36 | 0,65 | 0,77 | 3,35 |
| 2 | 2,92 | 0,64 | 0,46 | 6,45 |
| 0 | 0,74 | 0,66 | 0,66 | 3,32 |
| 0 | 0,77 | 0,92 | 1,51 | 1,44 |
| 0 | 1,70 | 0,49 | 0,90 | 4,08 |

Daten

Selbstorganisierende Karten

Daten-Vorverarbeitung

Kohonen-Algorithmus

DBT2   HMGA2   age   PPARG

Auswertung

Visualisierung

Exploration

Modellbildung

Statistik

Anwendungen

Neuer Patient

Auswertung

Diagnostik

Prognose

EP 3 714 067 B1

Fig. 1

$$w[j] := (w[j][1], w[j][2], ..., w[j][n])$$

$w[j][1]$ $w[j][2]$ ..... $w[j][n]$

$X_p[1]$ $x_p[2]$ .... $X_p[n]$

$$\text{Input}_p = (X_p[1], x_p[2], ..., X_p[n])$$

Fig. 2

EP 3 714 067 B1

Fig. 3

**Underfitting**
Hypothesenklasse ist nicht
ausdrucksstark genug

Wahrer Fehler

**Overfitting**
Hypothesenklasse ist zu
komplex

**Optimale
Komplexität**

**Komplexität**

Fig. 4

EP 3 714 067 B1

Fig. 5

Fig. 6

Fig. 7

Fig. 8

EP 3 714 067 B1

Fig. 9

Fig. 10

Fig. 11

EP 3 714 067 B1

Fig. 12

EP 3 714 067 B1

Fig. 13

# AdipoQ
# Segmentation

| Cluster | Beschreibung | abs Profilgr. | Anteil(%) | T2DM | HMGA2 | Alter | AdipoQ |
|---|---|---|---|---|---|---|---|
| C 1 | Nicht-Diab... | 0.41 | 44.26% | 0.00 | 1.21 | 64.7 | 1.12 |
| C 2 | T2DM ST1 | 0.98 | 21.31% | 2.00 | 3.48 | 76.4 | 0.74 |
| C 3 | T2DM ST2 | 0.93 | 16.39% | 2.00 | 1.19 | 66.3 | 0.67 |
| C 4 | Nicht-Diab... | 1.34 | 13.11% | 0.00 | 1.21 | 37.3 | 0.79 |
| C 5 | T2Dm ST3 | 2.05 | 4.92% | 2.00 | 1.01 | 75.3 | 1.79 |

Fig. 14

Fig. 15

Fig. 16

43

Fig. 17

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015208083 B3

**In der Beschreibung aufgeführte Nicht-Patentliteratur**

- **TOMIYAMA AJ ; HUNGER JM ; NGUYEN-CUU J ; WELLS C.** Misclassification of cardiometabolic health when using body mass index categories in NHANES 2005-2012. *Int J Obes (Lond),* 2016, vol. 40, 883-6
- **PEARSON J ; POWERS MA.** Systematically initiating insulin: the staged diabetes management approach. *Diabetes Educ,* 2006, (32), 19S-28S
- **SZMITKO PE ; TEOH H ; STEWART DJ ; VERMA S.** Adiponectin and cardiovascular disease: state of the art?. *Am J Physiol Heart Circ Physiol.,* 2007, vol. 292, H1655-63
- **HIRANO T ; YASUKAWA K ; HARADA H ; TAGA T ; WATANABE Y ; MATSUDA T ; KASHIWAMURA S ; NAKAJIMA K ; KOYAMA K ; IWAMATSU A et al.** Complementary DNA for a novel human interleukin (BSF-2) that induces B lymphocytes to produce immunoglobulin. *Nature,* 1986, vol. 324, 73-76
- **QUINLAN, J. ROSS.** Induction of decision trees. *Machine Learning,* 1986, vol. 1 (1), 81-106
- **QUINLAN, J. ROSS.** C4.5. Programs for machine learning. *J. Ross Quinlan. San Mateo, Calif.: Morgan Kaufmann (The Morgan Kaufmann series in machine learning),* 1993
- **QUINLAN, J. ROSS.** Improved use of continuous attributes in C4. 5. *Journal of artificial intelligence research,* 1996, vol. 4, 77-90
- **HASTIE, T. ; TIBSHIRANI, R. ; FRIEDMAN, J. H.** The elements of statistical learning: Data mining, inference, and prediction. Springer Verlag, 2001
- **SONQUIST, J.A. ; MORGAN, J.N.** The Detection of Interaction Effects. Survey Research Center, Institute for Social Research, University of Michigan, 1964
- **VERGLEICHE SCHWARZ ; GIDEON E.** Estimating the dimension of a model. *Annals of Statistics,* 1978, vol. 6 (2), 461-464
- **KLEMKE M ; MEYER A ; HASHEMI NEZHAD M ; BEIGE G ; BARTNITZKE S ; BULLERDIEK J.** Loss of let-7 binding sites resulting from truncations of the 3' untranslated region of HMGA2 mRNA in uterine leiomyomas. *Cancer Genet Cytogenet,* 2010, vol. 196, 119-123
- **LIVAK KJ ; SCHMITTGEN TD.** Analysis of relative gene expression data using real-time quantitative PCR and the 2 Method. *Methods,* 2001, vol. 25, 402-408